# EUROPEAN PATENT APPLICATION

(11) **EP 4 811 384 A1**
(43) Date of publication of application: **23.09.2026**
(21) Application number: 25165038.8
(22) Date of filing: 20.03.2025
(51) Int. Cl.: G16H 30/40, G16H 50/20, G16H 50/70

(54) **DETERMINING EFFICACY OF A PROCEDURE BASED ON USING DIFFERENT IMAGING MODALITIES**

(71) Applicant: Koninklijke Philips N.V., 5656 AE Eindhoven (NL)
(72) Inventor: HENDRIKS, Bernardus Hendrikus Wilhelmus, Eindhoven (NL); FERNANDO, Shakith Devinda, Eindhoven (NL); CLUITMANS, Matthijs Joseph Maria, 5656AG Eindhoven (NL); LUCASSEN, Gerhardus Wilhelmus, Eindhoven (NL); OLIVAN BESCOS, Javier, Eindhoven (NL); REM-BRONNEBERG, Debbie, Eindhoven (NL); WITHAGEN, Petrus, Eindhoven (NL); HUMMEL, Erik, Eindhoven (NL); VAN NIJNATTEN, Fred Simon Berend, Eindhoven (NL); VAN DE HAAR, Peter George, Eindhoven (NL); ELENBAAS, Thijs, Eindhoven (NL)
(74) Representative: Philips Intellectual Property & Standards

(57) **Abstract**

A system (CS) and related method for computing imagery. The system (CS) may comprise an input interface (IN) for receiving input data including an initial image obtainable by an imaging modality (IA1) of a region of interest, ROI, prior to a procedure performable in relation to the ROI. A predictor component (PC) computes a predicted virtual image representative of the ROI post-procedure. An output interface (OUT) provides the predicted image.

## Description

### FIELD OF THE INVENTION

The invention relates to a computing system, to an imaging arrangement, to a system for training a machine learning model for use in such a computing system, to a training data providing system for providing training data for training a machine learning model, to related methods, to a computer program element, and to a computer readable medium.

### BACKGROUND OF THE INVENTION

Cardiac afflictions of all manners are still the leading cause for premature death, degradation of quality of life, etc. As per an 11 June 2021 factsheet (available online at *https:*//*www.who.int*/*news-room*/*fact-sheets*/*detail*/*cardiovascular-diseases-(cvds)*) by the WHO (World Health Organization), the toll due to CVD (cardiovascular disease) stands at 17.9 million premature deaths worldwide.

Modern medicine provides a range of options, some of which are imaging based. For example, Coronary Computed Tomography Angiography (CCTA) is a well-established modality for dealing with CVD. It can be used non-invasively during the diagnostic and planning phase of percutaneous coronary interventions (PCI) for example.

In particular, CCTA can be used to create a three-dimensional reconstruction of the coronary vessels, to determine virtual fractional flow reserve (FFRCT), and to determine the optimal locations for stent placement, etc.

On the other hand, intravascular ultrasound (IVUS) is a catheter-based diagnostic modality used to view the inside of a coronary artery, providing a real-time view. With IVUS, the structure and composition of the arteries can be inspected, such as narrowing, plaque build-up, calcifications, bifurcations, sizing of the vessels, etc. It can be used to determine the effect of, for instance, stent placement interprocedurally.

In a workflow, when only IVUS is employed, an initial IVUS scan may be made (diagnostic scan), and the IVUS catheter is removed. After the treatment (for instance stent placement) is completed, IVUS is inserted again to make a post-treatment scan to determine an effect of the treatment. The initial, diagnostic scan is time consuming and is typically not performed in all vessels that can be scanned. This may hamper the treatment of the patient.
Conversely, when using CCTA, but not IVUS, the operator/user cannot assess the effect of the treatment. Furthermore, the FFRCT may not always be accurate.

### SUMMARY OF THE INVENTION

There may be a need for improved handling of multi-modal imaging support.

An object of the present invention is achieved by the subject matter of the independent claims where further embodiments are incorporated in the dependent claims. It should be noted that the following described aspect of the invention equally applies to the imaging arrangement, to the system for training the machine learning model for use in such computing system, to the training data providing system for providing training data for training the machine learning model, to the related methods, to the computer program element, and to the computer readable medium.

According to a first aspect of the invention there is provided a computing system, comprising:
- an input interface for receiving input data including an initial image obtainable by an imaging modality of a region of interest, ROI, prior to a procedure performable in relation to the ROI;
- a predictor component capable of computing a predicted virtual image representative of at least a part of the ROI post-procedure; and
- an output interface for providing the predicted image.

The region of interest may be part or whole of a system of conduits, such as, eg, a vasculature/vessel, urinary tract, gastro-intestinal, or in fields other than medical, part of a cave system, sewers, plumbing, hydraulic, etc.

The procedure may include effecting a modification of the ROI, such as a remedial action, or other.

In embodiments, the system comprises an assessment component capable of assessing an outcome of the procedure based on the predicted image.

In embodiments, the predicted system is so computed by the predictor component that the predicted image is capable of representing an effect of one or more devices usable in the procedure.

In embodiments, the predicted image, thanks to the computing by the predictor component, is capable of representing a planned effect of the procedure in relation to the ROI.

In embodiments, the input data includes any one or more of: i) data indicative of the, or a, device usable in the procedure, ii) one or more known characteristics of the ROI and/or of its surrounding.

The said surrounding may embed or enclose at least part of the ROI. In the medical field, the surrounding may be formed by at least a part of a patient/subject's body, an organ, tissue, etc. The subject may be human or an animal patient, such as in human medical or veterinary applications, respectively. Instead, the surrounding may be any other enclosure, such as a cave, or any other such surrounding that may preclude direct /unaided access of the said ROI, and thus may call for ionizing or invasive imaging means in some cases.

In embodiments, the assessment component is capable of so assessing further based on a post-procedure image acquirable by the imaging modality or by a further imaging modality.

In embodiments, the two imaging modalities are different, and have respective one or more sensors for acquiring respective measurement data, based on which the respective initial and post-procedure imagery is obtainable, and wherein the one or more sensor of one of the two modalities is situatable within or at the ROI during data acquisition, and whilst one or more sensors of the other imaging modality is situatable outside the ROI during data acquisition. Thus, the sensor may not necessarily be situated within the ROI, although it may well be, but may be instead situated at suitable proximity (depending on the sensor setup) to the ROI, either from within the surrounding or outside it, as per the particulars of the modality and as may be expedient in the circumstances.

The at least two different modalities may call for at least two different imaging apparatuses, one or each modality, but or may be practiced instead on the same imaging apparatus (which, to this effect, may be run in different modi), as needed.

In embodiments, the assessment component is capable of comparing the initial image and the predicted image.

In embodiments, the said comparing is based on spatially the initial image and the post-procedure image.

In embodiments, the predictor component is based on a trained machine learning model.

In embodiments, the trained machine learning model is of the generative type.

In embodiments, the two imaging modalities are medical.

In embodiments, at least one of the two imaging modalities is of the tomographic type.

In embodiments, at least one of the imaging modalities is non-ionizing.

In embodiments, at least one of the imaging modality is any one of X-ray based, optionally spectral, MRI or nuclear, and the other is any one of ultrasound, US, in particular IVUS, optical coherence tomography, OCT, photoacoustic, PA.

In embodiments, at least one of the imaging modalities is configured for facilitating spectral analysis.

In embodiments, the imaging modality includes a computed tomography, CT, imaging apparatus, and wherein the further imaging modality includes an intravascular ultrasound, IVUS, apparatus.

In embodiments, the ROI includes at least a part of a system of conduits at least partly inside the, or a, surrounding.

In embodiments, the system of conduits is at least a part of a vasculature in a subject.

In embodiments, the vasculature is a cardiac vasculature.

In embodiments, the procedure is one of treating a lesion in the vasculature.

In embodiments, the procedure is one of reducing a stricture in the system of conduits for improving flow of a fluid in the said system of conduits.

In another aspect there is provided an imaging arrangement comprising the system of any one of the above aspect or embodiments, and further comprising at least one of i) at least one of the at least two imaging modalities, ii) a display device capable of visualizing at least one of the initial image and the predicted image, or a result provided by the assessment component, iii) the assessment component, iv) at least one data memory on which is stored at least some trained parameters of the trained model.

In another aspect there is provided a system of training the machine learning model, based on training data.

In another aspect there is provided a system for providing the said training data

In another aspect there is provided a computer-implemented method, comprising:
- receiving input data including an initial image obtainable by an imaging modality of a region of interest, ROI, prior to a procedure performable in relation to the ROI;
- computing a predicted virtual image representative of at least a part of the ROI post-procedure; and
- providing the predicted image.

In another aspect there is provided a method of training the machine learning model as in any one of the above-mentioned aspects and embodiments, based on training data.

In another aspect there is provided a method for providing the training data for the said training method.

In another aspect there is provided a computer program element, which, when being executed by at least one computing system, is adapted to cause the computing system to perform any one of the above methods.

In another aspect there is provided at least one computer readable medium having stored thereon the program element, or having stored thereon at least some of trained parameters of the trained machine learning model.

The proposed setup allows efficient handling of such a procedure, in particular a procedure that is reliant on multi-modal imaging support. The proposed predicted image allows a kind of "indirect" cross-modality co-registration of the initial first modality (eg, CCTA) pre-op imag,e with the second modality (eg, IVUS, OCT, etc) post-op image, thus bypassing the need of a second modality pre-op image. This speeds up the procedure overall, reduces (in a medical setting) patient discomfort, and reduces wear-and-tear on potentially expensive imaging equipment with increased service life, reduced down-time and reduced frequency of procurement of replacement equipment, etc.

The proposed setup may be understood to address the problem of how to provide for certain procedures a good preplanning of the procedure, and/or provide a reliable treatment efficacy check, while keeping clinical workflow simple.

For example in some embodiments, the proposed setup allows efficient integrated use of two imaging modalities, such as IVUS+CCTA, which yields a full view of all arteries (thanks to CCTA), decreased procedure times (no need for a full diagnostic scan, and the possibility of navigating the IVUS catheter through the virtual CCTA coronary artery tree), and allows for intraprocedural or final treatment assessment (via IVUS).

Thus, what is proposed herein in some embodiments is to obtain a preprocedural image (such as via CCTA or via other modality) and use this to make a treatment plan for the patient. The preprocedural CCTA or other modality image is morphed into a virtual post-treatment image (e.g. of IVUS type modality for example, if this is to be used post-procedure). Eventually, the pre-operative CCTA data is then indirectly compared, via the virtual post-treatment image with an actually acquired post-treatment IVUS image. This comparison may be used to provide a quantitative measure of the efficacy of the treatment. In case the required target efficacy (such as in terms of effected changes in physiology, eg gained (enlarged) vessel lumen, FFR, etc) is not met, the procedure can be rerun, with modification(s), until efficacy of the treatment is established.

As a result of the proposed setup, in the cathlab only one IVUS pullback of the lesion has to be made (only after the treatment) reducing the complexity of the workflow in the cathlab. No pre-op IVUS is needed. With the CCTA, the complete coronary tree may be studied, and an overall outcome of the procedure can be assessed, as compared to IVUS, where only the local effect of part of the procedure can be assessed (eg, assessment of how well a stent is placed, etc). Said more generally, a combined use of global and local imaging modalities can be facilitated herein, harnessing the respective strength of each, thereby complementing each other in an efficient manner.

*"user"* relates to a person, such as medical personnel or other clinical user, operating the imaging apparatus or overseeing the imaging procedure. In other words, the user is in general not the patient. However, in autonomous imaging setups, patient may take over from or share some task(s) with clinical user.

*"subject"* is used herein in the general sense to include animate "subjects" such as a human or animal patient, or anatomic parts thereof, but also includes inanimate objects such as an item of baggage in security checks or a product in non-destructive testing. However, the proposed system will be discussed herein with main reference to the medical field, so we will be referring to the "subject" as "the patient" or a part of the patient, such as an anatomy or organ, or group of anatomies or organs of the patient.

In general, the term *"machine learning"* includes a computerized arrangement (or module) that implements a machine learning ("ML") algorithm. Some such ML algorithms operate to adjust a machine learning model that is configured to perform ("learn") a task. Other ML operate direct on training data, not necessarily using such as model. This adjusting or updating of the model is called "training". In general task performance by the ML module may improve measurably, with training experience. Training experience may include suitable training data and exposure of the model to such data. Task performance may improve the better the data represents the task to be learned. *"Training experience helps improve performance if the training data well represents a distribution of examples over which the final system performance is measured".* The performance may be measured by objective tests based on output produced by the module in response to feeding the module with test data. The performance may be defined in terms of a certain error rate to be achieved for the given test data. See for example, T. M. Mitchell, "Machine Learning", page 2, section 1.1, page 6 1.2.1, McGraw-Hill, 1997.

*"invasive"* in relation to an imaging modality does not necessarily mean that there is invasive action in respect of the ROI itself, although this may well be so such as in the PCI examples described herein. However, in other cases an invasive / internal imaging sensor may be placed in or at another organ or position in an anatomy instead, such placement being different from, but sufficiently proximate to, the ROI. Thus, "invasive" in general as pertains to imaging is to include herein an imaging modality, wherein the imaging sensor of such modality (either pre-op or post-op) is within a surrounding that at least partly encloses or embeds the ROI or proximate organ, region, etc. Thus, in the medical field of main interest herein, the term "invasive" may indicate that such sensor may reside inside subject (their body) during, and/or for the purpose of, such invasive/in-situ imaging of ROI or related region. Pathways to ROI or the place at which invasive sensor is placed may use natural openings or orifices, or such access may be otherwise provided

### BRIEF DESCRIPTION OF THE DRAWINGS

Exemplary embodiments of the invention will now be described with reference to the following drawings, which, unless stated otherwise, are not to scale, wherein:
Fig. 1 shows a schematic block diagram of an image-based arrangement IAR configured to support a procedure in respect of a region of interest;
Figs. 2 schematically illustrate cross sections of a tubular structure suffering from constriction, and an action of a device operating in such a structure to remedy or alleviate the construction;
Fig. 3 shows a block diagram of a computing system as may be used herein to support an image based procedure;
Fig. 4 shows a flow chart of a computer implemented method for supporting an image based procedure;
Fig. 5 shows a schematic block diagram of a training system that may be used herein for training a machine learning model for use of such trained model in the system of Fig. 3 or in the method of Fig. 4;
Fig. 6 shows a schematic block diagram of a generative type machine learning model and its training as may be envisaged herein in some embodiments; and
Fig. 7 shows a flow chart of a method of training a machine learning model.

### DETAILED DESCRIPTION OF EMBODIMENT

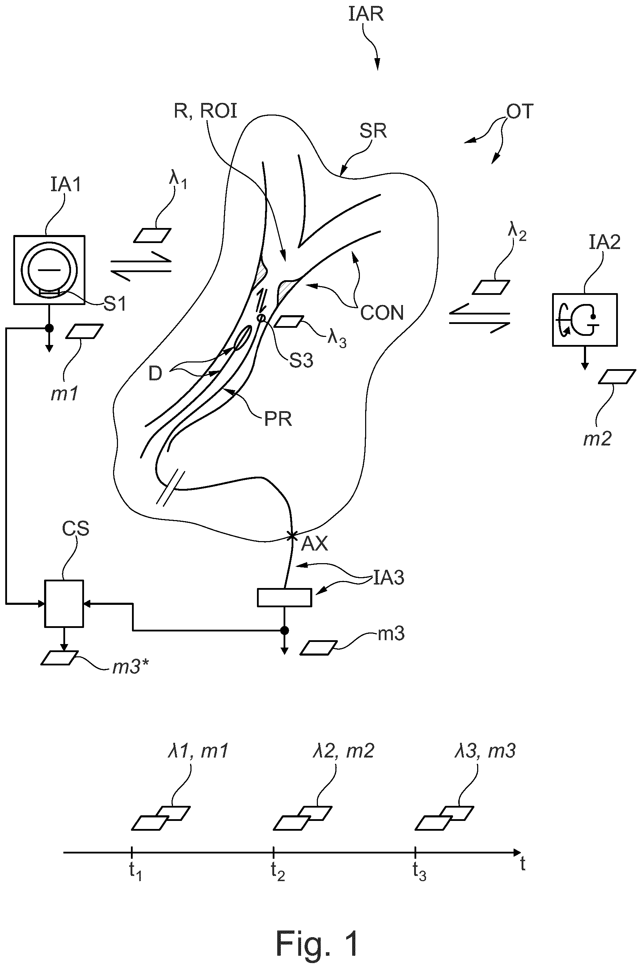

Referring now first to the schematic block diagram of Fig. 1, this shows an image-based arrangement IAR. The arrangement IAR provides image-based support for a procedure to be performed in relation to a region of interest (ROI) R. In the following, application examples of the arrangement IAR will be mainly drawn from the medical fields. In particular, the image-based procedure pertains, in preferred embodiments, to medical procedures, mostly interventional, but the below described principles may also be practiced in fields of endeavor outside the medical.

The imaging arrangement IAR provides such image-based support for the procedure based on at least two, in some cases three, imaging modalities. However, the below will mostly be focused on arrangements that use two such imaging modalities. Each imaging modality may be implemented by a respective imaging apparatuses IA1-IA3. Having said that, in some cases plural imaging modalities may be implemented by the same physical imaging apparatus, when operated in different modes, or according to different protocols, etc. The respective imaging apparatus may be operable to obtain respective imagery *m1, m2, m3* in relation to the region of interest R in respect of which the procedure was, is, or is to be, performed. Broadly, and as will be explained below in more detail herein, the arrangement uses a computing system CS that allows more efficient use of the various modalities, in particular of what will be described herein as a post-procedural imaging apparatus IA3. An efficient and reliable check of the procedure's success may be enabled by the proposed system CS.

Oftentimes the procedure is interventional. The procedure may pertain to a region of interest R within a surrounding SR. The surrounding SR may occlude the region R from unaided visual inspection or "eyeballing". Such may be the case for medical interventions in particular for example in relation to an inner organ, such as the heart. Certain cardiac procedures may be called for, such as a PCI (Percutaneous coronary intervention). Thus, the procedure may effect or facilitate remedial action in respect of at least a part of the ROI R. In the PCI example, the ROI R may include a portion of a cardiac blood vessel system CON inside the surrounding SR of human subject's body.

More generally, the region of interest R may be a portion of any system of conduits CON through which fluid may pass. The procedure may proceed over plural phases, such as navigation phase, vessel preparation phase (such as dilation via ballon catheter or other tool/device D in PCI), deployment phase (such as stent placement in PCI). and withdrawal phase of the device D, for example of the said balloon catheter or stent in PCI or other, in other medical or non-medical procedures.

Imaging apparatuses IA1, IA3, or indeed IA1-IA3, may be operable in respect of the procedure or phases thereof. For example, one imaging apparatus ("imager") IA1 may be operable prior to the procedure, to produce one or mor pre-procedural ("pre-op") image *m1.* Thus, this imaging apparatus may be referred to herein as the pre-procedural image apparatus IA1. Inter-procedural ("intra-op") images *m2* may be optionally provide by inter-procedural imager IA2. There may be an optional reconstructor (not shown) for reconstruction, but using herein solely projection imagery *m2* interprocedurally may be sufficient. Thus, such imagery *m2* may be provided during the procedure, such as for image-guided support of the procedure. And there may be post-procedural ("post-op") imagery *m3* obtained by post-procedure/procedural imager IA3. This is illustrated by way of timeline t at the foot of Fig. 1 proceeding over time periods *t1-t3.* Using the above terminology, the imagers IA1-IA3 may be referred to herein by extension, at times, as "pre-op", "intra-op" and "post-op" imagers IA1-IA3, respectively.

Generally, each imager IA1-IA3 may include a signal source for issuing forth an interrogatory signal that interacts with patient matter/tissue of interest, the signal being thus modified, and detected as modified signal (eg, as spatially varying radiation intensities, such as electromagnetic radiation signal or acoustic radiation signal, or other) at a suitable transducer/detector device (sensor) S1-S3 of imager IA1-IA3, respectively.

In more detail, the intra-op imager IA2 may be of any modality but is preferably arranged to allow acquiring intra-op imagery *m2* as a live action feed acquired as a time series (stream) during the ongoing procedure proceeding over one or more of the said phases. Location of one or more devices/tools D used in the procedure vs the local part of surrounding SR or ROI R may thus be displayed to user in display device, thus conferring said image-guidance. Thus, the live feed inter-procedural imagery *m2* may provide image guided support of the procedure performed. The procedure may be done using one or more devices D that are introduced into the surroundings SR in which the system of conduits CON is embedded or enclosed. Thus, if it was not for the imager *m2,* the device as such would be occluded from view. The inter-procedural imager IA2 may be an interventional imaging apparatus, such as an X-ray based C-arm. Such imager has particular structural features, such as a gantry with a cut-out (such as provided by its "C"- shape) that allows the user or interventionalist access to imaged subject (such as patient or mammal suffering from a heat infliction) to perform the procedure in respect of the region of interest, by using the one or more tool or device D, such as catheter(s), stent, guidewire(s) etc. As mentioned earlier, it is not a necessity herein that each of the imaging modalities envisaged calls for a different apparatus. For example, imagers IA1 and IA2 may be as such essentially the same physical apparatus, but may be operated in different modi to implement different such imaging modalities. For example, C-arm imager IA2 may be used in acquiring fluoroscopic projections intra-procedurally, and, prior to the procedure, the C-arm imager IA2 may have been operated in different mode, namely in 3D tomography mode, to acquire projections long a scan path around the ROI, for tomographic reconstruction into a tomographic volume or slice that may serve as the pre-op image *m1.*

The imager IA2, if used, is preferably non-invasive so is able to acquire the live feed imagery *m2* from the outside of the patient's body SR such as X-ray based projection data λ2 that is acquired and displayed as imagery *m2* on a display device in a "cath lab" for example for the interventionalist to consider. Of main interest herein is however the pre-op imager IA1, and the post-op IA3, or respective pre-op and post-op modalities more generally. Thus, the imager IA2 may be said to be interventional in the sense that it allows by its particular physical structure (eg, C-arm) access to subject during imaging for user to perform the procedure on subject. At the same time, the imager IA2 itself may be non-invasive as its sensor S2 (in this case, an X-ray sensor, eg of the flat panel 2D type having X-ray sensitive detector pixels) resides outside subject's body SR during imaging.

The post-op imager may be invasive type, but not necessarily in all embodiments. An ("internal") sensor S3 of the invasive post-op imager IA3 may be an array of transducers, such as in the case of an interventional vascular ultrasound system ("IVUS")as is indeed envisaged herein in embodiments. This sensor S3 resides inside the patient's body SR during acquisition of the acoustic measurements λ3 which may then be processed into ultrasound imagery, post-op imagery *m3,* to be used displayed herein as mentioned and as will be detailed below in more detail. The pre-op imager IA1 may be of the non-invasive type, but, again, not necessarily in all embodiments. An ("external") sensor S1 of the pre-op imager IA1, such as an X-ray sensor (preferably, flat-panel, of the 2D type or other) resides however outside the patient SR during acquisition of projection data λ1. The projection data λ1 (measurements of the detected intensities) may be acquired from plural directions of the region of interest or of other region, for example in a rotational, tomographic setup. Thus, pre-op imager IA1 may be a diagnostic CT scanner. The multi-directional projection data λ1can then be computationally processed by reconstructor into tomographic pre-op imagery *m1*, to be then consumed herein by the computing system CS, as will be described in more detail below. The imagery m1 may be displayed on a display device in order to assist planning the procedure.

The (non-invasive) inter-procedural imager IA2, if indeed used, is preferably X-ray based and provides projection views as captured by its sensor S2 of the region of interest. Such projection images *m2* are useful for some purposes but of course suffer from intervening structures which in such projection imagery are combined and collapsed into the same image values (line integrals). For at least this reason, the pre-procedural and post procedural imagers are used that are capable of providing more details, properly spatially resolved for better monitoring and in particular for better understanding in more detail of the initial state of ROI and assessment of the expected procedural outcome, respectively. To this end and as mentioned above, the modality IA1used for the pre-procedural imaging is preferably tomographic, and may indeed be X-ray based such as CT, but other tomography imaging modalities such as nuclear, MRI or other are not excluded herein. The, in general, invasive, post procedural imager IA3 may be also tomographic or not. If it is tomographic, it may include the ultrasound such as intervascular ultrasound IVUS, or may include any one of OCT, photoacoustic, or optical imaging, such as endoscopic imaging, or other. When intra-procedural imager IA2 is used, its imagery *m2* allows post-op imager IA3's sensor arrangement S3, which may appended to a probe PR, to be suitably visualized to allow introduction into the system CON of conduits, such as into blood vessel VS (ROI), which may be in need of treatment/procedure. In any case, the respective sensors S1 and S3 or pre-op and post-op imagers are configured to acquire respective measurement data λ1, λ3. In preferred embodiments, the pre-op measurements λ1 may be X-ray projection imagery preferably multi-directional as mentioned, whilst the post-op measurements λ3 are ultrasound readings, preferably also from different directions. The latter may be realized for example by rotating sensor S3 about its axis, or acoustic transducer array S3 may be arranged as a 360° array, as needed.

In one example of the procedure as envisaged herein, an angioplasty may be performed as one example of a PCI, in order to treat a stenosis. Such stenosis is a narrowing of a blood vessel, which in some cases may amount to a blockage of the vessel. The device D is introduced through an access point AX, such as an incision into a large enough vessel, and is then threaded to the region of interest under image guided support as conferred by the live action imagery λ2. However, use of such inter-procedural imager IA2 is optional herein, although envisaged in preferred embodiments.

Referring now to the pre-op imager IA1, this may use external sensors S1 (just like the inter-procedural imager IA2, if is used) to acquire non- invasively pre-op imagery of the region of interest prior to the procedure. Imager *m1* may be understood as an initial planning image that maps out the situation or condition of the ROI R "as is" pre-op. Pre-op imager IA1 is preferably of the tomographic type as mentioned. Such imager IA1 could be a doughnut shaped diagnostic CT scanner that scans the subject SR prior to a procedure, but may be instead MRI or nuclear, as needed.

The post-procedural imager IA3 on the other hand has a sensor S3 that is not external to the patient in use, but is designed, for the imager IA's operation, to be physically introduced into the subject SR, just like device D. Using such internal sensor S3 allows capturing live feed imagery from within the patient in-situ at the region of interest after the procedure has been performed by the one or more devices D. Such in situ imaging allows gathering better, more high-fidelity, data on the outcome of the procedure. This is in contrast to using imagery from a non-invasive imager, such as pre-op imager IA1, as such imagery may still allow for some interpretation or artifacts, whilst the risk for such to happen with the invasive post-op imagery *m3* is non-existent or low, at any rate lower than for imagery obtained by non-invasive imager such as imager IA1 for example. The post procedural apparatus IA3 may be an IVUS device or OCT, or any other. It may include the mentioned probe portion PR carrying at its distal end acoustic sensor S3. Part of the probe PR with its end having the sensor S3 is introduced into patient SR, at the same or a different access point. Sensor S3 is then equally threaded (like the procedure-effecting, or -facilitating, device D) towards the region of interest, so that the sensor S3 is proximal to the treated site, and is then operable to acquire invasive post-op imagery *m3* there of the ROI R post-procedure.

What is proposed here is the earlier mentioned computer system CS that processes the pre-op imagery *m1* and, optionally but preferably, the post-op imagery *m3,* to produce a result. Preferably, the result provides an indication on whether a pre-planned procedural target or goal in respect of the ROI R has been met when concluding the procedure. In more detail, and as will be explained below, the computing system CS is configured to produce a simulation *m3** of a post-op image without actually acquiring such image using sensor. The simulated image m3* represents a prediction of a configuration of the region of interest as would be expected if the procedure were successful. Said differently, the computing system CS may be operable to produce the simulation as a predicted image *m3*.* The predicted/simulated image *m3** is an approximation of an expected successful procedure. Oftentimes this predicted image m3* may be of interest herein in its own right. It may be displayed of otherwise brough to user's attention for training purposes for example, statistical evaluation other. Preferably however, and as envisaged herein in the main, the predicted image *m3 ** is combined with the actually acquired in situ post procedural live image *m3.* Specifically, the two images *m3**, *m3* are compared. The comparison may be suitably quantified, eg in terms of one more suitable metrics, and the result may reflect the outcome of such comparison. Such quantified comparison allows in an efficient and reliable manner assessing whether the procedure has actually concluded successfully and, if not, further action may be taken. The user may be notified by having the result based on such comparison result displayed, or by having this otherwise brought to the user's attention. In simpler embodiments, instead of computing such result r, essentially a score on the success of the procedure, the two images *m3, m3** are displayed, such as displayed on a screen of display device concurrently, for visual comparison by user. In some embodiments such concurrent displaying is caused in addition to computing the quantified result r, and this result r t may too be displayed or otherwise notified.

The proposed set-up allows avoiding unnecessary repeated imaging runs of invasive imager IA3. Earlier, the invasive imager IA3 had to be used more than once as described above in the section "Background", to check for outcome, which is cumbersome and time consuming. For example, in some such setups, the pathway through which the device D and the probe PR of the interventional imagery travels to the lesioned site ST are shared. It may only be large enough to be occupied by either device or probe, one at a time, but not both. Such pathway may be provided by a larger gauge transport catheter through which other devices, including smaller catheters or other elongated devices of lesser diameter are slid. So, in general, it is either a relevant device D, such as a balloon catheter, guidewire device or any other that is resident within the patient, or it is the probe PR/sensor S3 of the post-op imager IA3 that is so resident. Thus, both may needed to be introduced and re-introduced repeatedly in turns, which consumed time. This unlike with the proposed set-up, where only a single run of the interventional imagery IA3 may be needed, provided the intervention turns out successfully. Said differently, what is proposed herein is that the computing system CS essentially "morphs" the input pre-op imagery m1 into image *m3 ** that corresponds to the modality of the post procedure interventional imager IA3. Thus, pre-op imager IA1, which may be a different modality from post-op imager IA3, produces pre-op image *m1* at a fist modality (eg CT), and this image *m1* is then morphed into the simulated image *m3** at a different modality, such as into an interventional inter-vascular ultrasound image. Thus, image *m3 ** can be better compared with the actual in-situ acquired post-op IVUS image *m3,* in order to so more reliably assess the success of the procedure, even though different modalities are in use pre- and post-op.

In more detail still, what is proposed herein is that different imaging modalities/apparatuses IA1, IA3 are used for pre-procedure and post procedure, and that the computing system CS herein is operable to computationally transform, or "morph", the imagery *m1* of the pre-procedural imager /modality IA1into one that may have been acquired by post-procedural imager/modality IA3. Thus, the morphed-into image *m3 ** has the "look and feel" (in terms of image structure, semantics, etc) as if it was acquired with the same modality as used for acquisition of post-op image *m3* (in this case, imager IA3). This allows better, in a more robust manner, comparing the simulated image *m3** and the actually acquired post procedural image *m3.* The proposed setup allows establishing whether there is procedural success, with better efficiency, with fewer imaging runs by post-op imager IA3.

In addition, in preferred embodiments, the post-op simulated image *m3** is such that it represents the ROI in a state that corresponds to an expected success of the procedure. Thus, simulated image *m3** represents the ROI in a configuration that represents "how matters should look like". It represents the expected, planned, outcome of the procedure, whilst the actually acquired post-image image *m3* represents the ROI R "as is" post-op.

Whilst the above and below are mainly confined to the medical field such as the treatment of lesions in blood vessels (in particular, cardiac vessels but also others) and such is indeed mainly envisaged herein, the proposed principles can nevertheless be seen to be applicable to a wider range of applications beyond the medical realm. For example, such ex-medical applications may include image-based repair work or assessments of inaccessible pipe, plumbing or cave systems, of hydraulic tubing work, or any other. Also, whilst many types of modalities can be combined herein for pre- and post-po imaging, it is in particular the CT and IVUS combination that has been found to yield good results, in particular for intervascular procedures such as PCI, angioplasty, atherectomy, and others, such as heart valve repair, or others still.

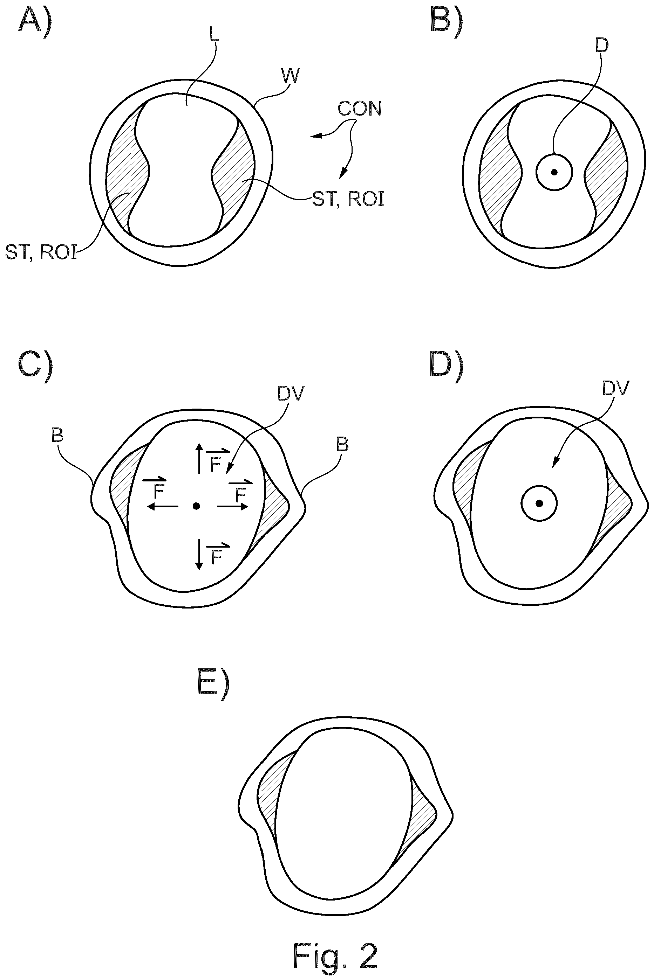

Before explaining operation of the computing system CS in more detail, reference is now made to Fig. 2, in particular Fig. 2A), which shows schematically a cross-section through a system of conduits CON as envisaged herein in embodiments. In particular, Fig. 2A) schematically refers to an example of a stenosed blood vessel, such as a vein or artery of the human heart of a patient SR, or a partial obstruction of vein in the head, that may cause patients to hear sounds that is has been found to be induced by turbulent blood flow (relevant for Tinnitus exams). Other such system of conduits that may be in need or intervention may include any other vessel or conduits, such as the urethra, a part of the intestine, bile duct, etc, depending on the procedure and case at hand. In this example of Fig. 2/2A, the surroundings SR are formed by the surrounding internal anatomy of the patient (various tissues, such as muscles, bones, other organs, etc) which enclose or a proximal to the vessels portion VS, CON of interest ROI. The view as per Fig. 2A) is one of a state of vessel VS pre-op, with an untreated lesion ST, such as a stricture (stenosis) forming ROI R. The view as conferred by Fig. 2A may correspond to a situation or condition as represented in the pre-op image *m1.*

The stricture ST is formed by the build-up of deposits, such as plaque, on the inside of vessel's (eg, an artery) wall W, thus reducing native cross section of vessel lumen L. Such deposits may be precipitated from blood passing through lumen L. Stricture ST may cause a reduction in blood flow which could hamper blood profusion of downstream tissue, which in turn could affect proper physiological functioning, and may lead to health problems for patient SR. The situation prior, during the procedure, and post- procedure is shown in cross sections Fig. 2B)-E), respectively, which are now described in more detail. In general, the cross-sectional views of Fig. 2 are taken across the longitudinal axis of the blood vessels in main direction of blood flow.

The view in Fig. 2B) shows schematically arrival or presence of the medical device D or tool to be used for performing the procedure. Such device D may be a balloon catheter as shown schematically in cross-section across its longitudinal axis as may be present after navigation to lesioned site ST, but prior deployment of such device. Thus, Fig. 2B) represents conclusion of navigational phase, where the device D, after induction into the patient SR at access point AX, has been successfully threaded to the lesioned site ST, ROI. The cross-sectional view in Fig. 2C) schematically illustrates deployment of the device D. In the illustrated case, such deployment may amount to expansion of the balloon body of the balloon catheter. Balloon body on expansion exerts a lateral force *̅F̅*̅ on the deposited material ST and thus displacing such material ST and urging same into vessel wall W from the inside. In response, wall W forms bulge(s) B that emerge on the distal/outer side of vessel wall. Now, with the deposits ST out of the way, the balloon catheter is then shrunk back as shown in the view of Fig. 2D). The outcome of procedure may be an enlargement of the lumen L, thus providing relief. Such relief is but one example of a result of remedial action as effected by the procedure. There may be other such remedial actions, depending on the nature of the application, case and procedure at hand.

At the final stage shown in Fig. 2E), the device D is retired leaving a treated site. At times the action of the balloon catheter as described is sufficient remedial action. At other times, after ballooning, a stent may need to be deployed and fixed in place in order to prevent wall W, and with it the displaced deposit, from contracting back and thus causing a relapse of stenosis ST.

What has been observed in particular in the above-described angioplasty procedure, is that some of the material that forms the stricture, such as plaque, is not only urged into the wall W of vessel VS, but may in fact break off forming debris. Such debris may then be washed away with the blood stream, or is aspirated or otherwise evacuated to avoid further complications , such as by using a suitable other device.

The view afforded by Fig. 2E) may in fact be one as may be produced by the computing system CS when morphing the pre-procedural image *m1* (eg, Fig. 2A) into the simulated post-op image *m3*,* this image *m3** being a prediction or simulation of how a successful treatment ought to look like and if it were acquired by modality corresponding to the post procedural imager IA3 actually used. Depending on whether the procedure results in a successful outcome, the view of Fig. 2E) may or may not correspond or be similar to the view afforded by the actually acquired post-op image *m3* by the post procedural imager IA3 for checking of procedure outcome.

Because of the simulated image *m3**, morphed from pre-op image *m1,* no invasive pre-op image is needed any more, thus saving one imaging run by imager IA3. Using non-invasive pre-op imager IA1 is sufficient. The use of invasive imager in the pre-op phase as was done in some existing protocols in the past is thus rendered obsolete herein, thanks to the proposed computing system CS.

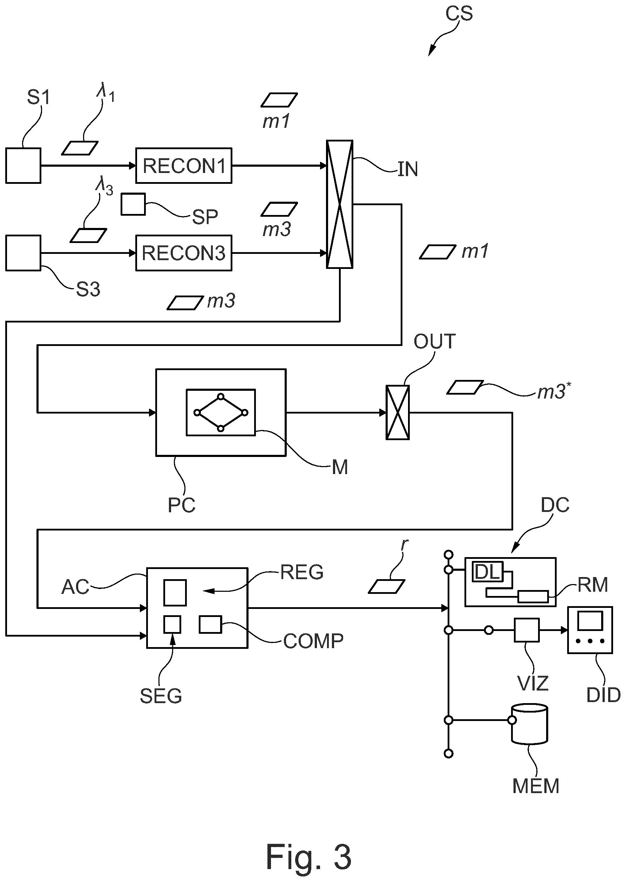

Reference is now made to the block diagram of Fig. 3 which shows components of the computing system CS in more detail. The computing system CS may be arranged, run on one or more computational nodes. Thus, computing system CS can be run on a single computing device, or can be run on multiple devices, eg in a Cloud based and/or distributed computing set-up, as needed.

Initially the pre-procedural image *m1* is acquired based on measurements λ1 acquired by sensor S1 of the pre-procedural imager IA1. As said before, such pre-procedural imager IA1 may be a CT imager where X-ray projection data λ1 are processed by a tomographic reconstruction algorithm RECON1 into tomographic volumes or slices *m1,* which image *m1* is received at input port IN of the computing system. The pre-procedural imager IA1 in so obtaining image(s) *m1* may run a contrast dye protocol. This protocol may ask for prior administration of contrast-conferring dye prior to acquisition of projection data λ1 to so confer better contrast to otherwise natively next-to-radiation-transparent structures, such as vessels or other soft-tissue. Thus, an angiographic CT arrangement (CCTA) may be used for imager IA1.

In addition, or instead, the pre-op imager IA1 may be capable or multi-energy acquisition for spectral imaging (dual energy, photon-counting, etc). Specifically, in embodiments, in addition, or instead of tomographic reconstruction, there may be an optional (further) processing of measurement data *λ1,3* by an optional spectral processor SP that is operable to compute material specific contrast imagery *m1*,*3* from the respective acquired raw data *λ1*, *λ3.* Such spectral processing yields spectral measurement data *λ1*,*3* from which spectral imagery *m1*,*2* may be reconstructed. Thus, a reference herein to "measurement data *λj*" or to *"*(*post*/*pre-op)* imagery *mj"* or to similar terms (*"projection data"*/*"image"*, etc) is to be constructed herein as a generic reference to either conventional, purely energy-integrated, data, or to such energy resolved spectral data. For example, the pre-procedural imager IA1 may be configured for X-ray based spectral imaging. In this technique, material specific contrast for pre-defined base materials can be obtained, as opposed to purely overall attenuation driven contrast as is the case for conventional energy-integrating-only X-ray imaging. In such spectral imaging, the imager IA1 may be configured for multi-energy image acquisition, where sets (at least two such sets) of at least two images at different acquisition energies (or energy "spectra") are acquired. Such sets are then combined computationally by spectral processor SP. To this end, spectral processor SP may implement a spectral algorithm to obtain such sets of material specific contrast imagery. For example, in this manner a "bone-only" image, a "contrast dye only" image, a non-contrast image, etc may be obtained. *R E Alvarez & A Macovski* reported such algorithm in their paper *"*Energy-selective reconstructions in X-ray computerized tomography", published in Phys Med Biol, vol 21(5), pp 733-44 (1976*).* Such or similar spectral processing may also be used, or may be used instead, by post-op imager IA3, or by intra-imager IA2, as needed.

The pre-procedural image *m1* is fed as input data *n* into a prediction component PC. Prediction component PC computes, based on the pre-procedural image *m1,* the said prediction *m3** image of the same modality as is to be used later by the post procedural imager IA3, and as representative of an expected success of the procedure. Thus, in PCI, the image *m3** may represent, when displayed, a view similar or corresponding to Fig. 2D) or E), with or without a suitable device present. Thus, in PCI, such imagery *m3** may represent, when displayed, vessel cross-section with enlarged lumen, with prior flow restricting deposits at least sufficiently removed or displaced out of the way. In some embodiments, and as detailed below, input data *n* may further comprise, in addition to image *m1,* contextual data c that indicates contextual feature(s) of the procedure, or other. The contextual data c may relate to any one of more of features of the post-op imager IA3 or of feature(s) of the device(s) D to be used for the procedure, and/or may relate to characteristics of patient and/or ROI in particular, such as bio-characteristics (BMI, sex, patient history). The context data c may be co-processed with image pre-op *m1* to produce simulated image *m3*.* The context data c may be assumed to be available in suitably coded form, such as a vector, matrix or tensor, etc. Embedding or coding algorithms (such as one- or many-hot-encoding) may be used to map raw context data into elements (vector) of a suitably dimensioned vector space.

The exact nature of the simulated/predicted image *m3** may depend on the imaging modalities IA1, IA3 used, and may differ from case to case. The predicted image *m3** may be made available through output port OUT and may be further processed herein. It may be provided, stored, visualized or otherwise used, such as displayed on display device DID by operation of a visualizer VIZ. Simulated/predicted image *m3** may be stored in memory MEM, or may be consumed by other one or more data consumers DC as will be described shortly.

In some embodiments, predictor component PC is machine learning (ML) based. Thus, the predictor component PC is, or may include, a trained machine learning model M, trained for the task of predicting such predicted image *m3** for representing ROI in the expected state, after successful procedure and in a representation that corresponds to the pre-op imager IA3's modality. In some such ML embodiments, the ML model M is of the generative type. Thus, the model M's operation may be understood as one of "repainting" input image *m1* in the style of the pre-op modality used, or to be used, for acquisition of the post-op image *m3.* Image structure of the simulated image *m3** may represent a structural state of the ROI in the event of a successful outcome of the procedure. The simulated "re-paint" *m3** in "post-op modality style" may be done before or after acquisition of the in-situ (eg, intravascular) image *m3.* The post-op imager IA3/modality is preferably of the invasive type. In any of the ML embodiments, generative or not, the prediction component PC may be based on the suitably trained machine learning model M that does the morphing or transforming of the input image *m1* into the predicted image *m3***.* Aspects of training may be described below in more detail.

Before or after computing of such image *m3*,* but after completion of the procedure, post-op imager IA3 is operable to acquire post-op image *m3.* Thus, in case of IVUS or other US modality, based on acoustic signature measurements λ3 as picked up by acoustic transducer S3 of imager IA3, an optional acoustic reconstructor RECON3 of post-op imager IA3 reconstructs post-op image *m3.* Acquired post op image *m3* is then likewise received at input port IN, or at another input interface, of system CS and is passed on to an assessment component AC for co-processing with simulation image *m3*.* Thus, in preferred embodiments, the assessment component AC ("assessor") is operable to assess, based on the predicted/simulation image *m3 ** and on the post-op image *m3,* a success of the procedure. The post-op image *m3 is* acquired after the pre-procedural image *m1* and preferably after the conclusion of the procedure. Optionally, the procedure is possibly done under image guidance through imaging apparatus IA2 (which is optional). The post-op imager IA3 may be acoustic radiation based such as in IVUS, but other modalities, preferably but not necessarily of the invasive type, may be used for imager IA3.

The result r as released by assessment component AC may represent a score, a metric or other numerical result, based on comparison of the two images, the simulated image m3* and the actually in-situ acquired post-op image *m3.* The assessment result *r* may be based on a comparison of the two images to measure the procedure's success. This is at least in part because *m3 ** is generated to represent a situation the ROI ought to look like in an event of planned for procedural success, whilst the in-situ image acquired *m3* represents the situation "as is" post procedure.

Both images *m3**, *m3* are processed by assessment component AC. Thus, assessment component AC may include a comparator COMP that compares the two images *m3 ** and *m3.* This comparison operation may include or may be based on in-image measurements and/ or other preparatory operations that assist the comparison. For example, operation of comparator COMP may optionally be based on, or may be assisted by, operation of a spatial registration component REG that spatially registers the (at least two) images *m3 ** and *m3.* The registration may result in the two images being aligned in a common spatial co-ordinate system to thereby facilitate comparison.

A segmenter SEG may be used to segment the images *m3** and *m3* to assist comparison and/or image registration, as needed, or to derive further metrics that may be or may not be used in the assessment, but may be instead displayed as addition information.

The assessment result r so released by assessor AC may be understood as a measure of an amount of discrepancy/divergence (if any) between images *m3**, *m3.* The assessment result *r* may be consumed by a data consumer D, possibly to inform further action. For example, in one embodiment the data consumer DC includes decision logic DL that decides whether the result *r* provided by the assessment component constitutes the success of the procedure or not. If it does not, flow control is passed to recommender module RM that may recommend the use of a different device and/or other action(s) that may be advisable in a re-run of the procedure, with better outlook for successful conclusion at that time. If the result does betoken success, processing of system CS ceases. In the latter case, system CS may remain mute and retire. Alternatively, the success of operation as assessed may be indicated by visual, sound or other clues for user's assurance.

Thus, the imaging apparatus IA3 in conjunction with the computing system CS may be operable in iterative fashion, until a successful result is achieved, with the recommender module RM providing respectively updated recommendations in each cycle on how to re-run the procedure, eg with different devices and /or different parameters etc to ultimately achieve success. When so re-running the procedure over one or more cycles, the system CS may use as input the pre-op image *m1* and the latest acquired post-po image *m3* from the current iteration cycle to predict the next predicted image *m3*ⱼ*, with *j* representing the respective procedure cycle. Thus, the original pre-op image *m1* may be "enriched" by updated image-based insight as provided by the latest prep-op image *m3ⱼ,* in case of non-successful procedure in the previous cycle j-1. Thus, the predictor component PC may take in as input 2-channel data comprising the original image *m1* and the latest post-op image *m3,* and both images are co-processed. The post-op image *m3* may be updated in the next cycle should this too result in a failure, and so on, until successful conclusion or until another stopping condition is met.

The recommender module RM itself may be arranged as a machine learning model separate from the predictor component PC. The decision logic DL may use a suitable policy, such as thresholding, based on one or more metrics that are computable by assessor in its assessment operation.

The assessment result r as such may be indicated, such as by being displayed on display device DID via operation of visualizer VIS, or such result *r* may simply be stored in memory MEM for logging or other purposes. Any one of the predicted image *m3** and/or of the result *r* may be displayed on the same display device as the interventional imagery *m2* is displayed on, or on a different device, as needed. All manners of visualization of the above image data *m3*, *m3**, *m1*, *m2* or result r either singly or in any combination of or any two or more of such image data and/or result r may be envisaged herein. For example, the pre-procedural image *m1* and/or the post procedural image *m3* may be displayed in addition alongside or alternately in sequence to the predicted image *m3** and/or the result *r* may be displayed in form of a suitable overlay graphics. The result r may be provided in numerical form indicative of the (image structural) discrepancy/divergence between the two image sets *m3** and *m3,* and thus of procedure success. In some embodiments, the result *r* may be in the form of the pixel/voxel-wise difference image |*m3-m3**|*,* or a function thereof. No recommender RM or decision logic DL function may be needed in some cases. The difference image may be displayed instead. It is the user who may then draw, based on their clinical experience, and in consideration of the difference image of other metric, their own conclusion on how (if at all) to rerun the procedure, whether there is success, etc.

The above described system CS admits for a range of modifications or refinements, any one or all of which are envisaged herein in different embodiments. Thus, the below describes various such modifications and refinements.

For example, pre-op image *m1* can be any one of CCTA, MRI, PET/SPEC. Pre-op image *m1* may be CB(cone-beam)CT such as may be done with the C-arm IA2, or other, reconstructed from frames acquired prior to the actual procedure. However, pre-op imagery *m1* may be instead IVUS, OCT, photoacoustic, or may even be some of the angiography images *m2.*

The acquired post-op images *m3* can be IVUS, OCT, as said, but may instead be angiographic images. If the pre-op is in-situ/invasive (such as IV), the post-image may not, or, if it is, the modality is different. Thus, in general, the modalities for *m1* and *m2* are different as this may provide a greater degree of assurance. However, if the pre- and post- treatment images are of the same modality (e.g. IVUS-IVUS, OCT-OCT), as such an option is not necessarily excluded herein, the registration between the images becomes simpler, as opposed to the case of different modalities such as CCTA vs IVUS. However, in this latter case, the cross-modality morphing by predictor component PC as proposed herein, preferably ML based, can be put to good use. Whilst in the above, pre-op modality was in general non-invasive whilst post-op is invasive, in some embodiments this may be reversed.

Other pre-treatment data which may be used alongside the pre-op image *m1,* and which is co-processed, may include certain context data c, which may be any data acquired before the treatment such as stress tests, ECG recording BMI etc.

In embodiments, the efficacy of the treatment (procedure's outcome, such as success/acceptability) can be estimated for one or more multiple phases of the procedure, rather than at the end of the whole procedure, and/or merely at the end of the last phase before conclusion.

While the pre-op images *m1* can be acquired before the patient enters the cathlab, IVUS *m3's* can be acquired at different phases of the procedure, for example at the beginning of the procedure (in IVUS, sometime called *"IVUSi"* image), or after the vessel preparation phase (sometime called "IVUSp" image), or, as mainly described herein, after the vessel treatment, such for example, when the lesion is stented (sometime called "IVUSt" image). Similar phased *m3's* can be defined for imaging modalities other than IVUS. By comparing against such *m3's* from different procedural phase IVUSi, IVUSp, and IVUSt, the progress success may be tracked. For example, the expected progressive gain in lumen diameter, or evolution of the procedure over its phase of any other parameter can be assesses in steps. For example, comparing against final IVUSt may define the total improvement (e.g. in terms of gained vessel lumen) of the PCI procedure. Instead, a comparison against IVUSp may give information on the improvement on the vessel gain due to the vessel preparation. Thus, in view of the "phased" post-op imagery *m3,* the term *"post-op* "as used herein may not necessarily refer to the completion of the whole procedure/its final phase, but may instead refer to the completion of any one of its earlier phase of the procedure, prior to the last. Thus, "post-op" may refer to time after, such as conclusion of, a given one phase of interest of the procedure. However, in general the term *m3* post-op may be used herein to indeed refer to the conclusion of final phase/he whole procedure (of a given cycle). But this is for illustration only, and does not exclude reference to conclusion of said earlier intermediary phase, prior to the final phase of the given procedure.

In the presented examples, the pre-op image may be a 3D-volume rendering of the patient anatomy such as in CCTA, whilst the *m3* in-situ image may be likewise tomographic-volumetric, such as in IVUS is an intravascular pullback. However, the proposed setup can also be practiced if a single cross-section post-op *m3* (such as single slice IVUS) is used, and/or from when from the tomography volume CCTA only a corresponding 2D vessel cross-section is selected. Hybrid processing, where slice is compared versus volume may also be considered in some embodiments.

As said, the virtual visual *m3 ** represents the anatomic configuration post treatment, in particular for a successful procedure or phase thereof. Thus, the virtual post treatment image *m3 ** includes information about the anatomy. Optionally, virtual image *m3** may further include image structure representative of one or more of the deployed procedural device D as resident therein, such as of a stent, inflated balloon, guide wire, or micro-catheters, without the need to specifically model these devices. Other methods, such as Finite Element Models, whilst not excluded herein, may require explicit laborious, and potentially error prone modelling in 3D. No such explicit device modelling may be needed herein when ML is used.

In an optional additional step, system CS may be operable to compute a virtual post-treatment anatomical model of the vasculature, based in one virtual *m3** visual. For example, the *m3** image may be segmented for vessel representation. This model may then be used to derive a virtual physiological measurement (such as iFR, FFR, or QFR) based on the vasculature model.

In some embodiments, some information from the pre-op image *m1* may be ported into the *m3*.* For example, in a spectral CCTA, info on calcium/plaque etc. may be extracted overlayed as additional information into the virtual image *m3*,* before and/or after the procedure. In this and similar embodiments, not only the virtual IVUS image *m3** is calculated from the pre-op CCTA *m1,* but also structures that, as such would, not be natively presented in the morphed into modality. Thus, the ported information may relate to features the pre-op modality is capable of imaging for, but not ordinarily the post-op modality. Thus, by this feature porting step the post-op modality is virtually endowed with additional imaging capability from the pre-op imaging modality, These additional features, such as extended calcified lesions (which can only partially be seen in IVUS or not at all), my then be added in the image *m3*,* but with instance in different rendering, such as shading, hue, or different color. Other such portable features may include plaque composition, plaque characteristics and FFR characteristics to mention a few. Furthermore, the IVUS image can be further extended beyond the penetration depth of IVUS. Apart from (spectral) CCTA specific information, also other pre-op information such as spectral CT or MRI information may be used to calculate the virtual IVUS image and to augment its native information content.

In some embodiments, in order to assess procedure success/acceptability (both terms can be used herein interchangeably), one may wish to determine whether for instance stent is placed as planned. The virtual stent placement as per the virtual image *m3 ** may be compared with the actual position of the stent as per the acquired image *m3.* Landmarking may be used by segmenting for same in the images *m3**, *m3.* For example, IVUS image *m3* is compared to the virtual IVUS image *m3** as morphed from the pre-op image *m1,* such as a CCTA. This allows to determine the position of the stent with respect to the planned position from the CCTA image. Based on this assessment, user may be informed about the actual position of the stent with respect to the planned position, e.g., in % -sensitive overlay (eg, 100% may code for a full match between actual and planned position, whilst 10% may indicate that only 10% overlap between the planned-vs-actual stent shapes). If required, the new (actual) position can be re-assessed in CCTA with a virtual FFR to determine whether the actual position provides sufficient therapeutic effect.

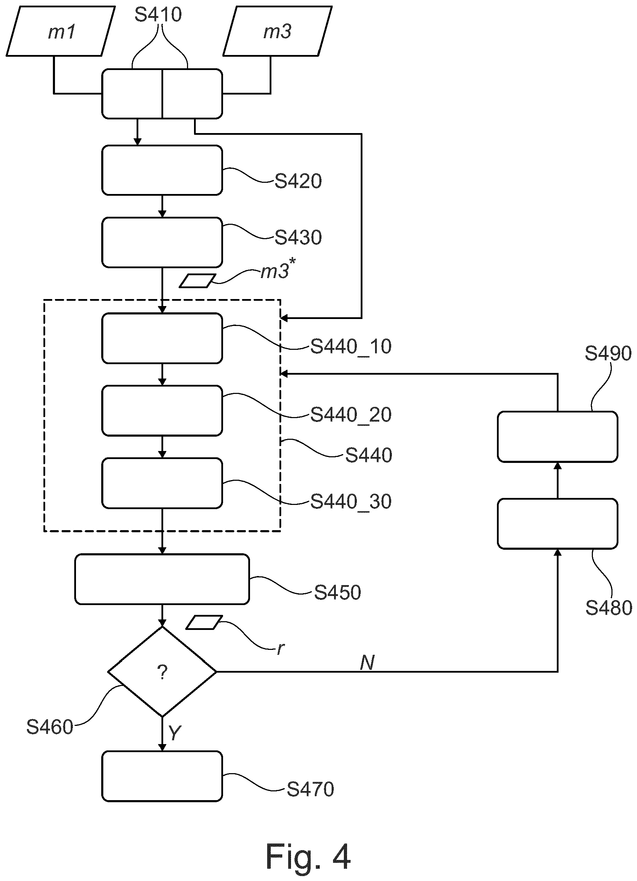

Reference is now made to the flow chart of Fig. 4 which illustrates a computer-implemented method. The method may implement the computer system CS as described above. However, it will be understood that the below described steps of the method are not necessarily tied to the architectures described above in connection with system CS, and may be understood instead as a teaching in its own right. The computing implementing method may be implemented by one or more computational nodes. The method facilitates image-based procedures, in particular in the context of multi-modal imaging. The method allows efficient handling, coordination, etc. of such multiple imaging modalities IA1, IA3. In particular, in relation to the image-based procedure, the method may be used to ascertain whether the procedure has concluded successfully and, if not, further processing steps may be suggested as mentioned earlier. The method may be thought of as a multi-imaging modality approach where imagery from different modalities are processed. In particular, the pre-op imagery *m1* from pre-procedural imaging apparatus IA1, and post-op imagery *m3* from post-procedural imager IA3 are processed herein, preferably are co-processed. The post-op imager IA3 is operative to acquire the post-op imagery *m3* after conclusion of at least one phase of the procedure or after a first run of the complete procedure.

In some embodiments, the pre-op image *m1* is a tomographic image such as a CCTA or other CT image acquired of a ROI of a subject in respect of which the procedure is to be performed. The pre-op image *m1* is acquired prior to the procedure commencing. At a later stage then the post procedural image *m3* is acquired by the different imaging apparatus/imaging modality IA3, such as an IVUS, OCT, or any other, as described above. At least one, or both, of the imaging modalities may be tomographic. At least one imaging modality IA3, IA1 may be of the invasive type. In embodiments, pre-op imaging modality IA1 and post-op imaging modality IA3 are tomographic. In such or other embodiments, the post-op imaging modality IA3 is invasive for in-situ, such as intravascular, imaging. The procedure may be a PCI procedure in respect of at least a part of a cardiac blood vessel. The procedure itself may be image-guided by operation of a third modality, such as by one of the imagers IA1, IA3, or by different imaging apparatus, the intra-procedural imager IA2, such as C-arm X-ray imager, or other.
Turning now to the method steps in more detail, initially, the pre-op image *m1* is acquired and then received at step S410.

The prep-op image *m1,* such as a CCTA (Coronary CT Angiography), enables medical user to understand patient anatomy, lesion morphology, and better prepare/plan procedure. However, by definition, such pre-op image *m1* on its own only provides a pre-operative "picture" of the patient anatomy, and user as such cannot use it to estimate the efficacy of the procedure. To this end, the post-op imagery *m3, m3** will be used as described above and as will explained below in the following steps.

Thus, at a later stage, after planning, and in particular after acquisition of the pre-op image *m1,* the procedure is performed in respect of the ROI, such as in PCI, angioplasty or any other. The ROI may be a stenosed site of a vessel.

The pre-op image represents in generally a larger volume, a larger field of view (FOV) than does *m3.* The *m1* volume/FOV includes the ROI. Eg, the whole heart may be imaged in *m*, whilst the ROI may be merely a section of a part of the cardiac vasculature. The later post-op image may be in general (in-situ) localized and its FOV relates only to a part of the volume (FOV) to which *m1* relates. Thus, in terms of voxels in 3D imaging domain, image *m3* is in general a subset of *m1*, *m3* ⊆ *m1.* The pre-op may be a single or few tomographic slices, or may a whole volume, and similar for post-op image *m3.*

At step S420 the predicted image *m3** is produced, based on input data *n.* The input data *n* includes the pre-op image *m1.* In some embodiments, certain structures of interest such as the deposit (eg, plaque such as Calcium, or other substance that causes the stenosis or relevant condition once wishes to remedy) is pre-segmented in *m1,* to better guide the prediction. It is the so pre-segmented *m1* that is then used as input to produce the predicted image *m3*.* Optionally but preferably, the input data *n* further includes context data c. The context data c may describe aspect(s) of one of more of the procedure, the ROI, the subject (patient), or other. Optionally, the contextual data is co-processed with the image *m1* in step S420. Such contextual data c may pertain to the particular type of interventional device D that was used or is to be used to perform the procedure, such as the nature, type or other specification(s) of the device that was used or is to be used. For example, the context data c may indicate that a balloon catheter is or was used, the model, make, gauge, or other specification. In other procedures than angioplasty, the context data may specify or indicate other catheter type or devices that may been used or will be used in performing the procedure. Including such context data c in relation to the procedure device to be used or that was used, may be beneficial. This is because the use of partial device, such as vessel preparation device D, will modify the plaque shape and morphology in different ways. Each device will have a different effect on the plaque. For example, non-compliant balloons are less aggressive than atherectomy devices, achieving a different level of modification.

The virtual image *m3** computed herein allows predicting the changes in the vessel and plaque structure, based on the vessel plaque as per pre-op (eg, CCTA) image *m1.* This is because the generated virtual image *m3** is expected to represent how the vessel will look after the procedure is run with the chosen device D as per context data c.

The context data c may indicate more than device, as some procedures call for different devices or different states of one or more devices, in different phases of the procedure for example. In addition, or instead, and more fundamentally, the context data may indicate the type of procedure itself. The procedure itself once performed may cause certain material/physical/physiological changes in the affected ROI, such as plaque deposits being pushed out of the way to increase lumen of the afflicted vessel section, an old valve being supplanted by an artificial one, and so forth.

Preferably, machine learning is used to effect step S420. Specifically, a trained machine learning model is used in step S420 to process the input data *n* and to produce, based on such processing, the predicted image *m3*.* As mentioned earlier, such predicted image *m3** provides, when displayed, a visual rendition indicative of such change for the event of a successful, planned, outcome of the procedure. Having said that, such processing S420 may be done instead, in alternative embodiments, based on analytical modelling, mesh modelling, FEM method, etc, with physically inspired simulation of the involved physical actions/forces (compression, shifts, debris formation, expansion of lumen wall, etc). Preferably however, machine learning is used as will be described below in more detail. A combination of ML based, and analytical modelling may also be envisaged herein.

This predicted "virtual" post-op image *m3** may include information about the treated anatomy and thus potentially, as a consequence, about the type, specification of the device(s), such as stents, inflated balloons, guide wires, or micro-catheters, etc, without the need for explicitly3D modelling such devices. Thus, as preferred herein, the input data includes the context data, in particular such device specification, in addition to the pre-op image input *m1*, as mentioned above.

At step S430, after such processing at S420, the predicted image *m3** is then made available for downstream processing. At this point or at any point after conclusion of the procedure, the post-op image *m3* is acquired and then received at step S410. The in-situ (eg, intravascular) image *m3** may be an IVUS image, acquired in a pullback or in any other way. The acquired *m3* may be one at one point in time, such as at the end of a phase other than the final phase such as end of navigation, vessel preparation, etc. An FFR measurement may also be done.

It is the received post-op image *m3* and the received predicted image *m3 ** that are then processed together at step S440. Such co-processing may include comparing the two images *m3, m3***.* This comparison may be driven by image structures as manifest in both images. The comparison may yield a result r' that quantifies the comparison. The compassion result may indicate the manner, degree, etc, in which the two images differ/diverge. Pixel-wise subtraction may be done to quantify such divergence. The comparison result may be based on, or may indeed be, the difference image that results from such a pixel-wise subtraction or other operation. Other, more involved metric/scores, other than, or in addition to, such pixelwise subtraction, may be used. For example, Euclidean distance, possibly squared, may be used in forming the difference image. The score/metric may be based on an *l^{p}* norm. The predicted image *m3** has preferably the same size (rows and columns of pixels) as the acquired post-op image *m3,* or such can be achieved by down-or up-sampling.

At step S450 the comparison result is assessed to yield an assessment result r. In this step S450, the result r' score/metric may be analyzed based on a policy. The comparing step S440 may be part of the assessment step S450.

The assessment result r or compassion result r', are passed on to follow-up step S460 to decide there whether, based on one or more of the results *r,r*' from any one or both of steps S450, S440, the procedure can be judged successful. If it can, the process terminates at step S470. If it cannot, the result *r,r*' is passed on to review step S480. In this step, based on the result and/or on the decision, one or modified aspects of the procedure to be repeated as suggested. The step S480 may use a look-up table or can be implemented in its own right as a separately trained machine learning model, trained to make such suggestions. Such model is different from the ML model that may be used in step S420.

If a new procedure is performed in modified form, whether based on suggestions at step S480 or simply based on the user using their medical knowledge in making modifications to the procedure by using a different device for example and/or using the same device in a different manner or in whichever way, a new post procedural image is acquired at step S490, and this is then compared with the predicted image *m3*,* and so on, with the method then repeating. Thus, the method may enter a loop, with repeated checks whether the procedure has concluded successfully, in order to iteratively arrive at some stage at successful conclusion of the procedure.

The following describes the above-mentioned steps in more detail. In particular, step S440 of comparing the two images *m3** and *m3* may include at step S440_10 an optional spatial registration of the two images. Such registration may not be needed, eg if motion can be neglected, or for other reasons. Optionally, this sub-step may be followed by sub-step S440_20 which includes a segmentation for structures such as vessel contours ("vessel tree"), etc., in at least one, or both, of images *m3, m3*.* At optional sub-step S440_30, based on the registration and/or segmentation, a vector field is established that describes how one of the images ought to be changed in order to be mapped onto the other image. This can be done by rigid and/or non-rigid transformations, such as any one of deformation, rotation, shift, or a combination of any two or all three of the foregoing. Indeed, deformations will often be required in some embodiments, in particular in the above-mentioned angioplasty embodiment, where there is a loss of volume incurred due to, for example, some debris breaking off from the plaque deposits or due to compression. Such disintegration or compression of deposited material may be due to action of the angioplasty balloon as it expands in deployment against vessel wall and the deposits there, as illustrated earlier, above at Fig. 2.

Referring back to step S450, the assessment may be based on variations in terms of the amount by which the lumen has been enlarged due to the deposits being removed, and/or other metrics such as stenosis cross section, virtual FFR (Fractional flow reserve) in-image measurement ("FFRi"), etc, based on vessel and deposit segmentations as described above. In cardiac applications, such as PCI, the deposit itself usually includes calcium. This insight can be harvested herein by using a spectral imager IA1 for acquiring the pre-procedural image *m1.* In such case, as mentioned above, multi-energy projection data λ1 is acquired and spectrally processed into ROI imagery *m1,* having calcium specific contrast, at the extension or reduction of attenuation effects caused by other materials that happen to be in the field of view of imager IA1 when acquiring projection data λ1.

Assuming a decent registration between the images *m3*, *m3**, any mismatch between corresponding structures within the two images may be done solely to procedure. Thus, procedural efficacy can be measured in terms of physiological improvement, such as gained vessel lumen and/or other similar parameters/metrics. For example, a comparison between the healthy inner-vessel lumen and the pre- and post- inner-vessel lumen can be computed, in order to estimate the treatment efficacy. The gained vessel lumen (luminal gain) can be computed between the inner-vessel lumen as per image *m3, m3*.* Other parameters can be utilized to estimate the treatment efficacy, such as the morphology of the plaque cross-section. Another parameter that can be estimated from the virtual image is vascular resistance or iFFR.

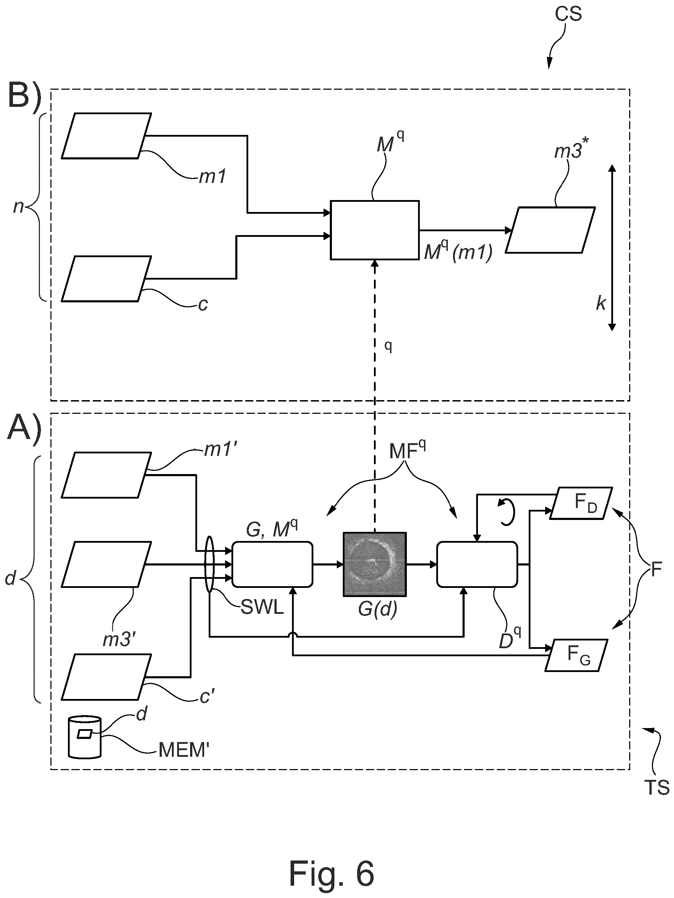

Some information from the pre-op image may be used, after segmentation, registration etc., between *m1* and *m3* or *m3***.* Using the lesion length estimate from the multiplanar reconstruction from pre-op image *m1* (such as the CT image) and the diameter and/or area stenosis from cross sectional CCTA and IVUS the vascular resistance R *~ L*/ *e⁴* (with *L* lesion length, and *e* lumen radius) can be calculated. This is in particular useful for deciding whether or not to treat a focal lesion with a significant diameter/area stenosis over a short length (hence a focal lesion) versus a more diffuse lesion with a less pronounced diameter/area stenosis over a significant longer length (diffuse lesion). Thus, in this case, a number of predicted images *m3***ₖ* is computed, based on different specifics of procedure, such as which device to use, etc. See Fig. 6B below for more details. Only on review of the various predictions are the specifics of the procedure decided on, and procedure may go forward. By predicting the result of a treatment (placing a virtual stent) in the virtual imagery *m3*,* such as in terms of vascular resistance combined with luminal gain yields, yet another parameter that can be checked using the post-IVUS pullback *m3* to confirm the actual treatment efficacy.

Referring back to step S480, which may include modifying/optimizing aspects of the procedure for repeated application is earlier procedure outcome in earlier cycle is judged not successful, such step may include suggesting use of another device (with different specifications, type), such as a vessel preparation device, another stent expansion, and/or using overlapping stents, or use of same device but at a different setting/state, etc. or any other as needed. In the later acquired image at step S490 after the modified procedure, this newly acquired post-op image may also be segmented for vessel contour, plaque contour, specific material (eg, calcium) - such as if spectral imaging mode is enabled, or any other as earlier described.

Describing now in yet more detail the comparing step S440, in particular to various in-image measurements and facilitator steps on which the comparison operation S440 may be optionally based, reference is now made again to registration sub-step S440_10. Such registration may be based identifying (segmenting) anatomical landmarks (e.g. vessel contours, plaque position, calcium) present in both, the acquired image pre-op image *m3,* and in the virtual pre-op image *m3*.*

Turning now to segmentation step S440_20, this may include segmenting for any one of more of outer-vessel contour, as well as inner-vessel contour, stenosis cross-section, calcium content and other anatomical structures in the *m3, m3*.* In contexts other than PCI, or angioplasty, segmentation for other strictures of interest in the ROI may be called for.

Turning now to image mapping step S440_30, this may include mapping corresponding structure from images *m3, m3** by displacement (eg, vector field) to bring about the alignment. The use of devices such as balloons, atherectomy devices, or the implantation of a stent will modify the plaque in several ways, for example by pushing the plaque into the vessel wall, or by changing the plaque's stiffness by breaking the calcium embedded in the plaque. The predicted image *m3** is expected to account for some of these expected modifications and is expected to represent an expected change in shape and morphology of the vessel and of the plaque material at the ROI. A match, if any, between expected post-op image *m3** and acquired image *m3* is therefore a good indication of success or lack of the procedure, especially if the specification of the actual procedure effecting device is also accounted for as indeed envisaged herein.

A guiding principle for the registration and/or mapping, which may allow formulating simplifying boundary condition, is assuming a certain invariance of volume. That is, whilst shape/morphology of the plaque deposit as represented in the imagery may change due to the result of the procedure, the volume of the plaque deposit as such may be assumed to remain constant. Whilst some debris may form (e.g. atherectomy), the loss of volume involved may be negligible in some such or other cases.

In order to facilitate the registration S440_10 it may be beneficial to spatially link the ROI position as per the pre-op image *m1* to the ROI position as per the acquired post-op image *m3.* Also, for the registering of *m3* and *m3**, spatial clues from the intra-procedural imager IA2, such as intra-procedural imagery *m2,* may be helpful as this may allow ensuring that the post-op image *m3* is taken at the location for which *m3** is computed.

To this end, clues from the intra-procedural imagery *m2,* such as angio- or fluoroscopy frames, as acquired by the C-arm IA2 or other such intra-procedural imaging modality. To this end, use of a biplane or dual source X-ray imager IA2 may be beneficial. For example, the precise location of the catheter D on the 2D X-ray image *m2* can be determined using an image segmentation algorithm. Due to the projective nature of X-ray, the location on the 2D image will not reveal the depth-component of the location of the catheter. In other words, it can be anywhere on a line between the determined 2D location on the X-ray detector and the focal spot of the X-ray machine. The depth- component can be determined in any one of more manners:
- by acquiring X-ray images from multiple angles, for example using said biplane system or said multi-X-ray source system (eg, "stereo tube");
- by linking the location in 2D to the corresponding vessel in the 3D image.
- by harnessing geometrical a priori knowledge on the devices involved: for example, in case of an asymmetrically shaped probe PR/device D, the depth can also be determined from a single 2D image.

As an alternative, or in addition to drawing positional information from the X-ray or other intraprocedural imagery *m2* as supplied by imager IA2, other locator mechanisms may be used, such as optical fiber-based shape sensing or other. For example, such positional information may be supplied instead by probe PR of the intra-imager IA3, when its tip or other reference portion arrived at ROI, such as when post-op image *m3* is acquired. The mentioned optical fiber-based locator may be used alongside IA3 (such as attached thereto) or the two systems may be used independently. In such or similar fiber-optical systems, a bundle of optical fibers is threaded through the system of conduits CON (eg, vasculature) and is thus forced into a characteristic shape at different locations along branches of the vasculature. This shape causes a location dependent characteristic diffraction pattern by a light signal that is send down fiber and as detected by a light sensor. The shape data can be correlated with the vessel tree segmentation in the pre-op image *m1,* thus effecting localization and at least partial registration. Thus, by comparing the curvature of the optical fiber to the curvatures of the coronary tree as extractable from the *m1,* such as a CCTA, one can determine the most likely vessel and position within that vessel. The accuracy of this determined position may be further improved by comparing the actual IVUS image *m3,* with ML-generated IVUS data from CCTA at the potential location.

Turning now in more detail to the above image generation step S420 for generating the predicted or simulated image *m3*,* this may be generated in a manner so as to resemble the imaging modality IA3 usable for the post-procedural image *m3.* Thus, in some embodiments, from pre-op CT input image *m1* the simulated an intra-vascular ultrasound image *m3 ** is estimated or morphed into. This output *m3** represents an the expected (estimated) structural configuration of the ROI. Ideally, in case of success of some degree, this estimated image *m3** ought to resemble in some approximation the actually acquired in-situ post-op image *m3.* The extent to which there is divergence between the two, may be thought to represent the degree to which there is success. If there is sufficient resemblance, in terms of image structure for example, an indication to this effect may be given to user such via displaying, sounding out a signal, etc. The degree to which there is resemblance/divergence may be quantified by a suitable score, metric, etc. as mentioned earlier above in relation to step S440. Such score, metric, or other quantification may be used to assess S540 efficacy of the procedure as explained above.

Whilst this morphing or simulation step S420 may be done explicitly, classically, analytically, such as by finite element methods on meshes for example derived from the input image *m1* and by using principles from mechanics, elasticity theory, geometrical methods, and so forth, such approach is, whilst not excluded herein, likely to be out-performed by machine learning based models. Thus, ML methods are preferred herein.

In ML approaches, the models there are not necessarily subjected to specific functional constraints to mimic some physical principles. For example, neural networks with at least one hidden layer of parameters are known to be able to approximate a wide range of functional relationships, given a large enough set of training data that implicitly represents such functional relationship. In neural network type ML models, there are very general and few a-priori assumption needed on the parameterization, such as filter coefficients, and their linear combination of inputs at one node as received from nodes' outputs from the preceding layer of nodes. In the present case, such functional relationship is believed to exist between the expected outcome as embodied by the generatable images *m3** on the one hand, and the ROI's initial state and configuration as representable by the pre-op imagery *m1.* This may be so because the initial structural status recorded by a given pre-op image *m1* is likely to some extent determine the outcome when the procedure is applied. In addition, if the nature, type, etc, of the treatment device *D* to be used in the procedure, to so effect the physical reconfiguration of the region of interest, this contextual information c together with the initial state *m1* may be correlatable to the expected outcome *m3***.* Thus, there may be a "latent" (as such - prior to training - unknown) mapping 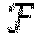 : 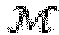 1 - 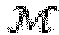 3 from the space 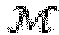 1 of pre-operative imagery to the space 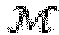 3 of expected physical configurations recordable in post procedural imagery in general. The model M may be trained to approximate this mapping 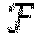 by model function *M*(*m1*)→ *m3** (that is, instances in post-training of model M applied to *m1* to yield *m3***)* by analyzing patterns in historical data *d=(x,y)* for such imagery, *x=m1', y=m3'.* Notation "y" may be used herein to indicate such historical post-op imagery that represented a historical successful procedure, whilst "x" pertains to associated pre-op imagery *m1'* as may have accrued in historical PCIs for example. In general, primed notation " ' " or such designators *"x"*, *"y"* may be used herein to indicate training data *d,* as opposed to un-primed notation for data encountered post training during use ("inference") by trained models in clinical practice or in ML model testing sessions. Another word on notation as used herein: the notation (x.y) for training data *d* is used herein purely for illustration and example, and is not to be construed in that the below described training methods or principles are necessarily supervised. They are not - although they may well be in some embodiments. Unsupervised, semi-supervised, reinforcement or other training setups are all envisaged herein in different embodiments for training model M for its prediction task.

In general, training data d may be sourced from medical databases, such as PACS or may be generated and collected over time in clinical practice at a medical site (hospital, radiology department, clinic, etc) in which the model M of system CS is intended to be used.
As proposed herein, the input *m1* based on which the trained machine learning model M is to compute the simulated expected outcome image *m3** may be understood as spatial data composed of spatially resolved values (pixels or voxels), just as the initial image *m1.* However, the values of which the simulated image *m3** is made up do not correspond to explicit physical measurements as do the values in the initial image *m1.* Instead, the spatially resolved values making up the generated image *m3** are pure estimates by machine learning models that operate on the physical measurements as recorded as values in the input imagery *m1.*

Preferably, it is not only the pre-op image *m1* that is processed by the machine learning model in training and inference, although this may be so in some embodiments. Instead, and preferably, the input data is of a multi-channel character. In other words, in addition to the image *m1,* contextual data c is co-processed by model therewith producing a yet better, more robust, estimate of the expected outcome image *m3***.* The generated image *m3** may also be referred to herein as the "outcome image" generally. Thus, the model M may be configured for co-processing of pre-op image *m1* and context data c into outcome image *m3*.* Thus, the input may be provided, stored, and processed as multi-channel data *(m1, c)*: *M(m1, c)* = *m3***.* The same applies to data used in training, and indeed, whatever is described herein in terms of the trained model applies equally to the model under training, expect that here, training data (*m1',c'*) is used as input to produce training output, based on which the training is done, as will be described below in more detail.

Because the processed data herein is generally spatial data, convolutional neural networks (either fully convolutional or at least partly convolutional) may be used herein as such models have been found to yield particularly good results with such spatially correlated data.

The input data in training, testing or inference can be arranged in matrices of two or more dimensions for the input data *m1.* The context data c may also be represented as a matrix or vector, such as may be obtained by one hot- or many-hot-encoding, or any other encoding scheme, such as extraction of latent representation from inner layers of an autoencoder network for example. Thus, encoding data may itself be achieved by using other machine learning models. In general, for example a specification of which device D to be used may be mapped into elements of suitably dimensioned vector space, such as may be done by embedding algorithms or other data preparation algorithms. Different coordinate components of such a vector may then represent the different device D types, or other context data such as ROI characteristics, patient bio-data, etc. More than one vector spaces may be used with different vectors ci from such spaces representing different such context aspects, such as device D types, or other context data such as ROI characteristics, patient bio-data, etc. ,may be used. Thus, using index j to represent such aspect, the input data may be formalized, stored, and processed as (*m1*,*c1*, *c2, ... , cj...).* Thus, entries in the so encoded context data c may represent stand for any one of bio-characteristics of the patient, patient history and/or as said for the type of interventional device useable in the procedure. Vectors *cj* may be combined into matrix 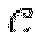 , thus yielding (*m1,* 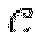 ). Embedding algorithm may ensure that a meaningful distance function may be defined in the respective vector space. Computing operations used in training and/or inference may be based on evaluating such distance functions.

Any other data formatting of the data *n, d* to be processed herein by model is envisaged. Formatting such data into matrix/tensor and/or vector form may be advantageous in particular (but not only) when using neural network type models, as indeed envisaged herein. Computing operations involved in training and/or inference, such as forward- and/or back-propagation) and/or various gradient based operations, may be implemented at least in parts as dot-product or matrix multiplication operation(s). This allows harnessing SIMD (*"Single Instruction Multiple Data*") setups, or other parallelizing techniques. In such cases, training and/or inference may be implemented efficiently on computing nodes equipped with multi-core computing chips (CPU), GPUs (graphics processing units), etc. Thus, such processors may be used by system CS and/or by training system TS (to be described in more detail below) which may be different computation entities.

The NN model M is made up of a set of computational nodes arranged in cascading layers, with nodes in one layer passing their output as input to nodes in a follow up layer. There may be an input layer where data *m1*, *m1'* is received and an output layer where model output such as *m3** after training, is output. The model M may be said to have a deep architecture because it has more than one hidden layer in between output layer and input layer.

The layers of the network, and indeed the input and output imagery, and the input and output between hidden layers (referred to herein as feature maps), can be represented as two or higher dimensional matrices ("tensors") for computational and memory allocation efficiency.

Preferably, the hidden layers include a sequence of convolutional layers, represented herein as layers. The number of convolutional layers is at least one, but a plurality is preferred, such as 2-5, or any other number, for example in the 10s or in the 100s or higher still.

The layers of the network, and indeed the input and output imagery, and the input and output between hidden layers (referred to herein as feature maps), can be represented as two or higher dimensional matrices ("tensors") for computational and memory allocation efficiency. Preferably, the hidden layers include a sequence of convolutional layers, represented herein as layers L₁ - L*_{N}*. The number of convolutional layers is at least one, but a plurality is preferred, such as 2-5, or any other number, for example in the 10s or in the 100s or higher still.

Preferably, some or all of the layers are convolutional layers, that is, include one or more convolutional filters which process an input feature map from an earlier layer into intermediate output, sometimes referred to as logits. An optional bias term may be applied by addition for example. An activation layer processes in a non-linear manner the logits into a next generation feature map which is then output and passed as input to the next layer, and so forth. The activation layer may be implemented as a rectified linear unit, or as a *soft-max-function*, a sigmoid-function, tanh-function, or any other suitable non-linear function. Optionally, there may be other functional layers such as pooling layers or drop-out layers (not shown) to foster more robust learning. The pooling layers reduce dimension of output whilst drop-out layer sever connections between nodes from different layers.

The NN model may be configured for multi-channel processing. For example, the model M may be configured to process a tensor including the input pre-op imagery *m1* and the context data c. Convolutional operators may operate separately on each channel, or preferably, there is cross-channel convolution to combine information from the two information sources, the pre-op image *m1,* and the context data c. The NN model principles described herein apply equally to the inference and learning/training phase, the difference being that in the latter case training data is applied to propagate through the network, whist in the earlier case data other than training data is used.

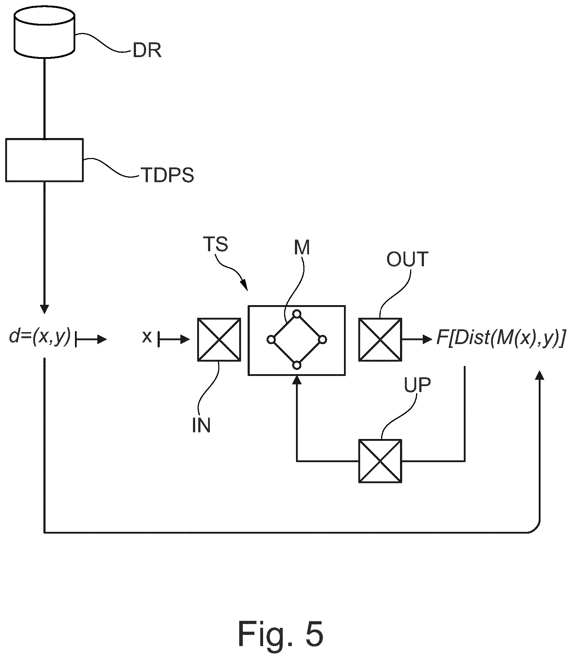

Before providing more details on training, reference is made first to Fig. 5, which shows in general a training system TS. The training system TS may include one or more computational nodes/entities, in generally different from the one or more computing node used for the computational system CS in inference. However, in some embodiments, inference, and training in respect of model M is done in turns by the same computational one or more entity. This may be advantageous in some clinical settings where model M is occasionally retrained in light of new training data that may accumulate during clinical practice. Thus, the system CS/TS may be switches between training and inference mode, as needed and appropriate.

In training phase, an architecture of a machine learning model M, such as the shown CNN/NN network as described above is pre-populated with initial set of "weights" which may form one example of learnable parameters *θ*, that is, those parameters that are changed/adjusted in the training. Weights *θ* of an NN type model M may represent a parameterization M*^{θ}*. The weights may include filter parameters of the convolutional operators. It is an object of the training system TS to optimize and hence adapt the parameters *θ* based on the training data *d.* In other words, the learning can be formulized mathematically as an optimization scheme where an objective function F, such as a cost function, is minimized, although the dual formulation of maximizing a utility function may be used instead. Assuming for now the paradigm of a cost function F, this measures the aggregated residue(s), that is, the summed error incurred between data estimated by the model M and some targets as per the training data *d=(x.y):* ${argmin}_{θ} F = {\sum }_{k} Dist \left[{M}^{θ}\left.\left({x}_{k}\right)\right),{y}_{k}\right]$

In eq. (1), function *M*() denotes the result of the model M applied to training input x. The result will differ in general from the associated target *y*. This difference, or the respective residuals for each training pair i, are measured by a distance measure *Dist*[.] Thus, the cost function *F* may be pixel/voxel-based, such as the *L¹* or *L²*-norm cost function, or any other norm *L^{p}.* Specifically, The Euclidean-type (such as squared or other) cost function in (1) (such as least squares or similar) may be used when the model is configured for a regression into an estimate for the virtual post-op image. The formulation in (1) is merely one example. More generally, objective function F is a function of the training data d and of model output M^{θ}(), and thus, at least implicitly, of the parameters θ, that is F ~*F*( *d, M^{θ}*), Also, the objective may not necessarily be one of minimization as in (1), but instead may be one of maximization, or indeed of partly both, such as in *minmax-* or *maxmin-* formulations. A *minmax-*setup will be described below with example reference to GAN type generator ML models. Also, in particular in some generative ML setups, such as GANs, the function F may refer only implicitly to data. For example, statistical formulation over the data d may be preferred, where the parameters may refer to a generator mechanism and/or related probability distributions underlying the training data, and/or the data generated by the model *M(d)* in the course of the training. Thus, in such generative setup, but also in others, summation in (1) may be formulated instead as one of cross-entropy or Kullback-Leibler divergence or similar, where it is probability distributions that are compared.

Various numerical setups may be used to solve (1) or the above described variants (maximization, or minmax, etc). In this connection, an updater UP may adjust/update the parameters to improve objective function F. For example, in particular in NN based models, updater UP may implement forward- and backward propagation or similar gradient-based techniques. Examples for such gradient-based optimizations may include gradient descent, stochastic gradient, conjugate gradients, Nelder-Mead, Maximum Likelihood methods, EM-maximization, Gauss-Newton, and others. Other Approaches are also envisaged, such as Bayesian optimization, simulated annealing, genetic algorithms, Monte-Carlo methods, and others still.

The training data may be provided by a training data provides system TDPS. This may take any one or more of many forms. For example, this may include a computing system for visualizing historical training data which may then be annotated by a human expert. The expert may use to this end a computer-supported annotator tool, such as computer mouse in interaction with visualization software. Additionally, or instead, training data provides system TDPS may be setup as database query tool or scrapper that searched medical image databases, such as PACS or other for suitable training pre-op imagery *m1'* and associated eg, invasive or other, training imagery *m3'* from another or same modality of the ROI of interest after the procedure or relevant phase thereof, and optionally for context data *c'.* The query may specify the ROI of interest, the type of procedure for which the model M is to be trained for The query should be run to ensure a sufficiently varied set of such data *d=(m1', m3',* [and optionally] *c'*) is returned, covering a diverse range of patients with differing bio-characteristics and health records, and tools D used and representative of a good mix of positive (procedure successful), and negative examples (procedure not successful).

Thus, ML models M, whether or not of the CNN/NN type, and envisaged herein include regression models where the input image *m1* is regressed into the outcome image *m3*.* Preferably (but not necessarily), and as will described in more detailed below, generative type machine learning models are used in some embodiments. Such generative type of ML models can be understood in terms of probability distributions that are thought to govern the distribution of the processable data. Broadly, generative ML models, once trained, are capable of producing new instances of data (different from the training data on which the model was trained) but that yet appears as if they have been drawn according the same, or at least sufficiently similar, probability distribution that underlies the training data. Some generative ML models operate based on randomized sampling. The given training data and the generated data may be conceptualized in terms of statistics as drawn samples from such probability distributions. In general, generative type ML models are trained to learn the joint probability distribution *P(X,Y)*, whilst general classifier models learn the conditional probability *P(Y*|*X)* (the probability of *Y* given *X*). Using the definition of conditional probability or Bayes' theorem, it is possible to learn *P(X*|*Y)*, or main interest herein, assuming marginal *P(Y)* is known. In this context, *X, Y* represent random variables defined over suitable sample spaces as training data instance data space X and associate label Y, respectively. Generative ML models use sampling from *P*(X|*Y)* or *P(X,Y)* to generate instances of samples from space *X*, even they such sample were not part of training data set. The training data set itself is considered a collection of samples from underlying data space *X.* At times it may be possible to sample from *P(X*|*Y)* without learning *P(X,Y).* Generative ML may be of the implicit or explicit type. In the explicit type, the relevant probability distributions are accessible in parameterized form, such parameters being part of the ML model parameters to be learned. When implicit, the relevant probability distributions are as such not accessible, but so may certain computational parameterized data generator mechanism, such as in GAN type models, that are known to be related to the probability distribution of the data space. In general, whether implicit or not, parameters are learned in a numerical optimization setup, which may be iterative and/or driven by gradient of cost function, in line with (1) above. The cost function is framed so that the parameters learned result in a probability distribution, from which the generated data is assumed drawn, that is similar to the true (target) probability distribution of the data. To this effect, the cost function may be based on the Kullback-Leibler ("KL") divergence, or other distance measure capable of measuring similarity between probability distributions, such as (binary-or general)-cross-entropy. The numerical procedure for finding the parameters may at times be framed as a maximum likelihood estimator ("MLE"). Randomized sampling from the relevant probability distributions may be numerically intractable as these may high dimensional data. Techniques from statistical physics, such as simulated annealing, Gibbs' Sampler, Metropolis-Hastings, etc, may be used to make the computations more tractable. The labels *Y* may be binary such as "*1*", *"0"* which may be taken as an indication on whether a given data can (eg, "*1*)") or cannot ("*0*") be considered a sample according to the relevant probability distribution.

Due to computational efficiencies, Generative type ML models M may be envisaged herein may any one or more of GAN ("Generative Adversarial Network(s)"), variational auto-encoders, diffusion type model, or other. Either one can be used herein but it has been found that GAN type models are of particular applicability for the set-up proposed herein.

Reference is now made to Figs. 6 which schematically shows a block diagram of a training system TS that is based on a generative ML model set-up, such as a GAN type set-up. In more detail, Fig. 6A) shows the training set-up for such a GAN model, whilst Fig. 6B) shows the inference or testing of such model *M*^{θ}, once its parameters θ are learned.

Turning first to training as per Fig. 6B), the training system TS uses the training data *d* as may be held in one or more memory MEM'. This training data d may include actual, historical, in clinical practice generated pre-op imagery *m1'* and, in addition, associated for the respective procedure, real acquired post-op imagery *m3',* and, optionally, associated context data c'.

GAN set-ups use a multi-model approach where the model to be trained is best thought of as a model framework MF parametrized by *θ*. Specifically, the model framework MF set-up includes two "sub"-models, a generator ("sub"-)model ) G, and a discriminator model DS, each suitably parameterized. Both are trained jointly and are interrelated in one provides input to the other. Once training concludes, the generator G may be released as the model *M^{θ}* sought herein. The discriminator is of lesser interest herein and may be discarded or may be re-used in follow training session, if needed. The discriminator model DS is different from the generator model G, both having its own set of parameters *θ_{G}, θ_{DS}* that together make up the whole parameter set *θ* =( *θ_{G}, θ_{DS}*)*.* Thus, each of the "sub-models" G, DS may be chosen as convolutional networks in their own rights, each having their own weights *θ_{G}, θ_{DS},* respectively. Model architectures other than NN or CNN may be used for either or both of models G,DS. Model G may be regressor, whilst model DS, as its name implies, may be a classifier. As mentioned, models G, DS are jointly trained on the training data. The cost function F for driving the training of the model framework MF comprises two parts, a partial cost function *F_{DS}* for the discriminator, and the partial cost function *F_{G}* for the generator G. Both partials are combined into the overall GAN training function F. The cost function may be based on a suitable distance measure, such as cross-entropy, Kullback-Leibler measure, or maximum likelihood. The probability distributions involved in the GAN setup is the one of the real, original data, and the distribution according to which generator outputs its data. A GAN cost function F type is one that is configured to urge for the generator output probability distribution to approximate the probability distribution underlying the training data. The training for adapting the parameters of the framework MF (that is, of parameters *θ_{G}, θ_{DS}*) can be expressed as minmax optimization problem in which the interplay between generator G and discriminator DS are conceptualized as two-player zero-sum-game. The iterative GAN training is one where there is convergence expected towards a Nash equilibrium, a concept from game theory. GAN setups are reported by Ian Goodfellow et al in "Generative Adversarial Networks", published online in 2014 with preprint archive arXiv, under publication identifier arXiv:1406.2661 (2014*)*, with the paper being available at the time of writing at *https:*//*arxiv.org*/*abs*/*1406.2661.* Many GAN types cost functions (or more generally, objective function) F have been proposed since the original objective function *F =* (*F_{DS} ,F_{G}* ) =: *V(θ_{G}, θ_{DS})* proposal in the *Goodfellow* paper (see their eq(1) on page 3 in the Goodfellow paper), such as Least Squares GAN function, Wasserstein GAN function and others in order to improve or speed up convergence. See for example table 1 on page of the paper by M Lucis et al, titled "Are GANs Created Equal? A Large-Scale Study", available, at the time or writing, on the arXiv preprint server under identifier arXiv:1711.10337 at https://arxiv.org/abs/1711.10337. Any such types of GAN objective function and others are envisaged herein. In general, the GAN loss function is based on the two probability distributions (of the generator action and the one underlying the data d) and (binary) cross-entropy or KL divergence, or functions or variations thereof. Actions underlying the mentioned conceptual two-player zero-sum game between models G, DS are two-fold and interrelated: The iterative adaptation if the parameter is such that it drives the generator towards configuration where its ability to fool the discriminator improves. The objective is to have the generator produce more and more realistic looking fake imagery, which the discriminator fails to distinguish from real samples *m3'* drawn from the training data set *d.* The parameters of the discriminator are adjusted in the training for a different objective, namely, to consistently tell the two classes, fake and real imagery, apart. The objectives are opposed, hence the convergence of the parameter set toward the Nash equilibrium.

In operation, generator G, based on a random seed z for example, provides output *D(z).* A random switch SWL may be used to selectively feed the discriminator DS with training input x. Depending on random selector SWL, training input x is either a sample from the training data d (in particular a random drawn sample *m3'*) or the generator output *D(z)*, Discriminator DS then attempts to correctly classify the presented data x as fake (say label "1") or as real (label "0"). Thus, DS wishes to classify whether x ∈ *d* (real "1"), or x *∉ d* (fake).

Whilst the classical GAN approaches preceded from random seed z as input for generator G, what is proposed herein instead is a data-driven regularized GAN variant, where the generator G input is in fact not random, or not only random, but is multi-channel to include as data-based regularizer the associated pre-op imagery *m1* ' and, optionally, context c' (in particular, type of device D used in procedure). Thus, *x* = *m1* ' or optionally x = *(m1 ',c').* Thus, the historical data, including pre-op imagery *m1'* and/or context data c' are co-processed by the GAN model's generator, whilst the discriminator processes, at least at times, based on *m3'* for classification purposes. In other words, the GAN networks generator G may be conditioned (conditional GAN, "CGAN") by having the generator accept *m1'* or *(m1',c')* as input, as opposed to, or in addition to, a random seed. A type of CGAN approach was described by M Mirza et al in "Conditional Generative Adversarial Nets", published by arXiv preprint, under identifier arXiv:1411.1784 [cs.LG], available online at https://arxiv.org/abs/1411.1784*.*

Generally, as proposed herein, the training system TS may proceed in iterations to improve the cost GAN function F, by updating *θ*, based on any suitable numerical optimizer, eg as mentioned above, such as cost function gradient ( *gradF_{θ,}* )-based, or any other. Thus, updater function UP for GAN may be based on any numerical optimization scheme, preferably gradient based, such as the mentioned gradient descent, stochastic gradient, etc.

Once trained, that is, once a stopping condition is fulfilled, the discriminator network DS as such is no longer needed (it may discarded or retained for optional follow up training cycles once new data emerge), and it is the generator network G that is then released, with its current set of parameters *θ*_{G}, as the model M *= M^{θ=θ}_{G}* to be used by the predictor component PC. The stopping condition may be set based on various policies, such as maximum number of iterations, cost function behavior, thresholding for changes in parameters with iteration step, etc. Thus, moving, now to the upper portion of Fig. 6, Fig. 6B that is, in deployment, testing or validation, a new pre-op image *m1* (not part of set d) is applied to the, now trained, model M, optionally in conjunction with the associated context data c, which data(*m1* or (*m1,* c)) is then (co-)processed by mode M to generate (infer) post-op image *m3** that represents the structural representation of the expected successful outcome. As said, the optional data c represents the type, specification etc, of the device D used or to be used in the procedure, and/or bio-characteristic of the patient or ROI in particular, or any other relevant contextual data which may have a bearing in outcome.

Whether or not GAN type model is used, not one but plural such generated images *m3 *ₖ* may be generated, one or more for each type *k* of device D*ₖ* to be used. Thus, index *k* may relate to the device (its spec, type, etc) to be used in the procedure as per input data *cₖ(Dₖ).* Thus, user can effectively "pre-sample" in advance different scenarios, for example by having the respective simulated imagery *m3*ₖ* displayed. User may then choose the device type that appears to yield the preferred outcome, as user may judge based on visual inspection of the displayed simulated imagery *m3*ₖ* Some or all of imagery *m3*ₖ* may be displayable, at least at times, concurrently to facilitate such visual inspection.

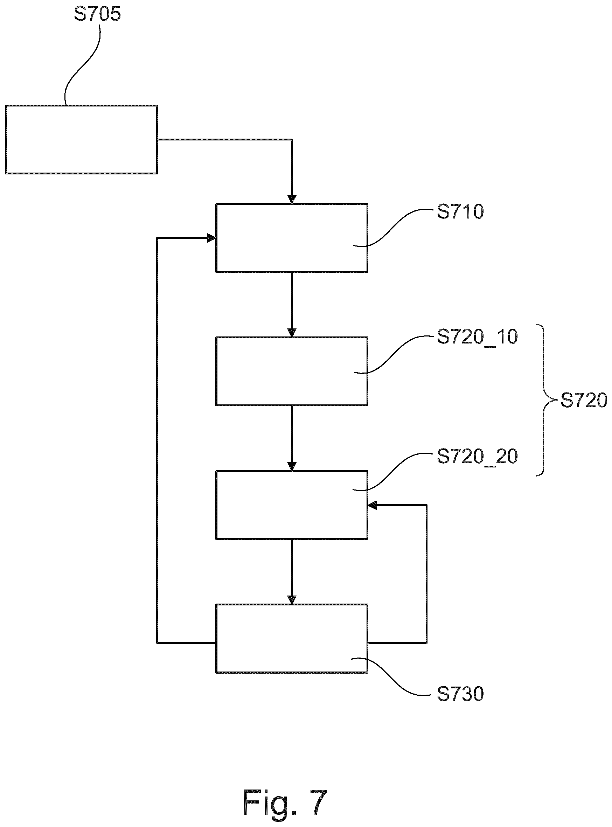

Reference is now made to Fig. 7 which shows a flow-chart of a computer-implemented method of training a machine learning model, in particular of a model as described above for use with the predictor component PC.

At step S705 the training data is procured and provided.

At step S710 the training data is received.

At step S720 parameters for the model are adapted, possibly over one or more iterations, based on the training data and objective function F (eg, (1) or other). The step S720 may include applying S720_10 training input data to model (at current parameter) to obtain model output. The model output may be compared S720_20 with other data (eg, targets), such other data items of the training data, or with other data such as generated by other mechanism of the training setup such as in GAN or other multi-model training frameworks or architectures. Steps S720_10, S720_20 may be function of objective function. Steps S720_10, S720_20 may include forward- and/or backpropagation operations, respectably. The adapting may be driven by a gradient of the objective function. In general, an objective of the training is to adapt the parameters of the model (possibly in one or more iterations) so that the objective function improves, and at step S730 the trained model is then made available for use in inference or testing, such as when a stopping condition has been met. This training condition or stopping condition can be based on the behavior of objective function (cost/utility) over iteration steps. For example, once function F drops under a threshold or as the case may be exceeds such threshold, training terminates, or training terminates once a preset number of iterative steps have been exhausted. Once training terminates, the current set of parameters that the model has will then be considered as the parameters of the trained model and the model can be released including those parameters for use in deployment (inference) and/or training/validation or other use. Principles described above in connection with Figs. 5,6 and eq (1) may be used for the said training method of Fig. 7.

As mentioned earlier, the model may be trained to represent the expected result *m3** not necessarily for the end of the final phase of the procedure, but for different phases s of the procedure *m3**ₛ*.* In this case, the training data may include suitable prior data m3'ₛ representative of ROI configurations as per the end of the respective phase s, such as navigation phase, vessel prep phases, etc, in addition to the final deployment phase. Different models Mₛ may be trained, one or more per such phase s. Thus, *"the model M"* for use herein in step S420 or by predictor PC, may be in fact a "bank" of models {Mₛ}, at least one such model Mₛ for each phase *s*. Alternatively, a procedural phase s indicator is part of the training data, in particular of the context data c'.

The above-described principles on ML learning are of equal application to the recommender step S480 or recommender module RM and its use of an appropriately trained ML model. Thus, training of such a model for step S480 /recommender module RM can be done as above, but with training data now being compiled as *x= m3"* and *y* = representative of an x-associated indication of the procedure/device type, etc modifications, suitable encoded as vector for example, again using embeddings such as 1-hot encoding or other. A classifier or regressor model may be used for this. The target *y* on modification associated with x can be provided by a human expert upon review of historical or synthesized ROI image data *x=m3*", representing outcomes that are not as planned (unsuccessful/unacceptable outcomes).

The components of the system CS may be implemented as one or more software modules, run on one or more general-purpose processing units PU such as a workstation associated with any one of the imagers IA1, IA2, IA3, or on one or more server computers/nods associated with a group of imagers.

Alternatively, some or all components of the system CS may be arranged in hardware such as a suitably programmed microcontroller or microprocessor, such an FPGA (field-programmablegate-array) or as a hardwired IC chip, an application specific integrated circuitry (ASIC), integrated into the imaging system IAR In a further embodiment still, the computing system CS may be implemented in both, partly in software and partly in hardware.

The different components of the computing system CS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it, which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs (set in brackets "()" ) in the claims should not be construed as limiting the scope.

## Claims

1. A computing system (CS), comprising:
an input interface (IN) for receiving input data including an initial image obtainable by an imaging modality (IA1) of a region of interest, ROI, prior to a procedure performable in relation to the ROI;
a predictor component (PC) capable of computing a predicted virtual image representative of at least a part of the ROI post-procedure; and
an output interface (OUT) for providing the predicted image.

2. System of claim 1, comprising an assessment component (AC) capable of assessing an outcome of the procedure based on the predicted image.

3. System of claim 1 or 2, wherein the predicted image is capable of representing an effect of one or more devices (D) usable in the procedure, and/or wherein the predicted image is capable of representing a planned effect of the procedure in relation to the ROI.

4. System of any one of the previous claims, wherein the input data includes any one or more of: i) data indicative of the, or a, device (D) usable in the procedure, ii) one or more known characteristics of the ROI and/or of its surrounding (SR).

5. System of any one of the previous claims 2-4, wherein the assessment component (AC) capable of so assessing further based on a post-procedure image acquirable by the imaging modality (IA1) or by a further imaging modality (IA3, IA2 ).

6. System of any one of the previous claims, wherein the two imaging modalities (IA1, IA3) are different, and have respective one or more sensors (S1,S3) for acquiring respective measurement data (λ1, λ3), based on which the respective initial and post-procedure imagery is obtainable, and wherein the one or more sensor of one of the two modalities is situatable within or at the ROI during data acquisition, and whilst one or more sensors of the other imaging modality is situatable outside the ROI or surrounding during image data acquisition.

7. System of any one of the previous claims, wherein the assessment component (AC) is capable of comparing the initial image and the predicted image.

8. System of any one of the previous claims, wherein the predictor component (PC) is based on a trained machine learning model (M).

9. System of any one of the previous claims, wherein at least one of the two imaging modalities is of the tomographic type, and/or wherein at least one of the imaging modalities is non-ionizing, and/or wherein at least one of the imaging modality is, or is based on, any one of X-ray based, optionally spectral, MRI or nuclear, and the other is, or is based on, any one of ultrasound, US, in particular IVUS, optical coherence tomography, OCT, photoacoustic, PA.

10. System of any one of the previous claims, wherein the ROI includes at least a part of a system of conduits (CON) at least partly inside the, or a, surrounding (SR), and/or wherein the system of conduits (CON) is at least a part of a vasculature (VS) in a subject (PAT) and/or wherein the procedure is one of treating a lesion (ST) in the vasculature.

11. An imaging arrangement (IAR) comprising the system of any one of the preceding claims, and further comprising at least one of i) at least one of the at least two imaging modalities, ii) a display device (DID) capable of visualizing at least one of the initial image and the predicted image, or a result provided by the assessment component, iii) the assessment component, iv) at least one data memory on which is stored at least some trained parameters of the trained model.

12. A computer-implemented method, comprising:
receiving (S410) input data including an initial image obtainable by an imaging modality (IA1) of a region of interest, ROI, prior to a procedure performable in relation to the ROI;
computing (S420) a predicted virtual image representative of at least a part of the ROI post-procedure; and
providing (S430) the predicted image.

13. A method of training the machine learning model as in any one of claims 8-11, based on training data, and/or a method for providing the said training data for such training method.

14. A computer program element, which, when being executed by at least one computing system, is adapted to cause the at least one computing system to perform the method as per claim 12 or - 13.

15. At least one computer readable medium having stored thereon the program element of claim 14, or having stored thereon at least some of trained parameters of the trained machine learning model as per any one of claims 8-14.
